# EUROPEAN PATENT APPLICATION

(11) **EP 4 596 712 A1**
(43) Date of publication of application: **06.08.2025**
(21) Application number: 24155636.4
(22) Date of filing: 02.02.2024
(51) Int. Cl.: C12Q 1/6806, C12Q 1/6855, C12Q 1/6869

(54) **METHODS OF IDENTIFYING AND USING BIOMARKERS**

(71) Applicant: WOBBLE GENOMICS LIMITED, Edinburgh, Lothian EH4 2HS (GB)
(72) Inventor: KUO, Richard Izen, Edinburgh, EH4 2HS (GB)
(74) Representative: Boult Wade Tennant LLP

(57) **Abstract**

Methods for producing an RNA vaccine for a subject with a disease and producing a database of disease biomarkers are provided. Methods for discovering disease biomarkers and diagnosing disease are also provided. The present invention enables frequent monitoring of subjects and adjustment of treatment. The present invention also provides methods for preparing processed nucleic acid samples with a more uniform distribution of sequences, which can be more readily analysed. The methods may be combined to further improve the ability to produce RNA vaccines and discover disease biomarkers.

## Description

### FIELD OF THE INVENTION

The invention relates to methods for analysing nucleic acid samples including methods for identifying and using biomarkers. The invention also relates to methods for producing an RNA vaccine, producing a database of disease biomarkers, and methods of diagnosis, prognosis and monitoring of disease.

### BACKGROUND

Screening for diseases can come in many forms, and recently there has been increasing interest in molecular screening approaches. The detection of RNA that is specific to cancer in a simple blood test, for example, has the potential to provide a readily acceptable and relatively non-invasive screening modality, as well as a prognostic biomarker (Larson, M. H. *et al.* A comprehensive characterization of the cell-free transcriptome reveals tissue- and subtype-specific biomarkers for cancer detection. 1-11). However, the handling and processing of samples for RNA sequencing is a challenge in the clinical setting. Additionally, developing a method that is sufficiently sensitive and specific has proven to be a challenge.

RNA expression is characterized by a wide range of expression levels across unique genes. There are a set of highly expressed genes known as housekeeping genes. These genes do not provide useful information, but they typically make up more than half of the total quantity of RNA in any given sample. In blood, this problem is even more extreme as globin RNA and ribosomal RNA typically represent over 95% of the total quantity of RNA (Harrington, C. A. et al. RNA-Seq of human whole blood: Evaluation of globin RNA depletion on Ribo-Zero library method. Sci. Rep. 10, 1-12 (2020)). Thus, a significant challenge with current detection methods is to achieve a breadth of detection for the overall transcriptome of blood.

A further problem is how representative the data is of the actual complexity and biological variations. While there are several RNA detection assays, until recently these methods only allowed for the detection of small fragments of each RNA molecule. This is problematic because each gene can be transcribed into a multitude of different RNA isoforms. This is possible due to the multiple combinations of transcription start sites, termination sites, and alternative splicing (Harrow, J. et al. GENCODE: producing a reference annotation for ENCODE. 7, 1-9 (2006). These alternative transcripts often have distinct functions and are often associated with specific cell and tissue types. As a result, the detection of small fragments of RNA alone does not tell the whole story.

### SUMMARY OF THE INVENTION

The invention is based on methods that take advantage of the ability to generate full length sequences from RNA extracted from blood without fragmenting RNA or cDNA products before sequencing. This provides a transcriptome representing any RNA that makes its way into the circulatory system. These include RNA from typical blood cells like red blood cells, white blood cells, other immune cells, etc. These would also include any other cells that are typically uncommon in the circulatory system such as cancer cells or cells that somehow dislodged into the circulatory system. This also includes extracellular RNA which could have originated from any cell within the body. By detecting RNA from all these sources and at full length the invention enables each RNA to be ascribed to a cell/tissue of origin as well as a state of cell or tissue behaviour. The transcription start site, end site, and splicing are features which typically represent unique combinations used by different cell types. This information can also be used to directly identify the protein isoform that would be translated from messenger RNA. The ability to detect protein isoforms and identify those that are associated with a disease allows for the development of treatments such as RNA vaccines.

It should be borne in mind that the various aspects have been devised so as to be advantageously combined and all such combinations are envisaged within the scope of the invention. It should also be appreciated that options described in relation to one area of improvement will apply *mutatis mutandis* to other areas; e.g. sample types, diseases etc. as appropriate.

The invention provides a method for analysing a nucleic acid sample, the method comprising:
(i) processing the nucleic acid sample; and
(ii) sequencing the processed nucleic acid.

The nucleic acid sample may be obtained from a biological sample of any suitable form including any material, biological fluid, tissue, or cell obtained or otherwise derived from a subject. The nucleic acid sample may be obtained from (cancer) cells or genetic material (DNA or RNA) derived from the (cancer) cells, to include cell-free genetic material (e.g. found in the peripheral blood). The nucleic acid sample may be obtained from a biopsy sample, optionally a solid biopsy sample and/or a liquid biopsy sample. The nucleic acid sample may be obtained from biological fluid or a fluid or lysate generated from a biological material. The nucleic acid sample may be obtained from blood. The nucleic acid sample may be obtained by extracting RNA from a biological sample (e.g. blood) obtained from a subject. cDNA may then be synthesized using the RNA as a template (i.e. by reverse transcription).

Blood samples may be readily and frequently obtained, allowing for repeated and non-invasive sampling of a patient.

The invention provides a method for analysing a nucleic acid sample, the method comprising:
(i) providing an RNA sample extracted from a blood sample obtained from a subject;
(ii) processing the RNA sample, optionally comprising synthesizing cDNA using the RNA as a template; and
(iii) sequencing the processed RNA or cDNA.

The blood sample may be whole blood.

Sequencing the processed RNA or cDNA generates a sequencing output. The sequencing output may provide a transcriptome representing any RNA in the blood. Thus, the sequencing output from the methods defined herein may comprise sequences of RNA from blood cells, other cells dislodged into the blood, and/or cancer cells as well as extracellular RNA. The sequencing output may be used to ascribe each RNA sequence/molecule to a cell or tissue of origin and/or to a state of cell or tissue behaviour.

The invention provides a method for analysing a nucleic acid sample, the method comprising:
(i) providing a nucleic acid sample comprising an RNA molecule, optionally wherein the nucleic acid sample is extracted from a blood sample obtained from a subject;
(ii) processing the nucleic acid sample comprising the RNA molecule, optionally comprising synthesizing cDNA using the RNA molecule as a template; and
(iii) sequencing the processed RNA or cDNA, wherein the sequencing output is used to identify the cell type and/or tissue type from which the RNA molecule is derived.

The invention provides a method for analysing a nucleic acid sample, the method comprising:
(i) providing a nucleic acid sample comprising an RNA molecule, optionally wherein the nucleic acid sample is extracted from a blood sample obtained from a subject;
(ii) processing the nucleic acid sample comprising the RNA molecule, optionally comprising synthesizing cDNA using the RNA molecule as a template; and
(iii) sequencing the processed RNA or cDNA, wherein the sequencing output is used to identify the protein isoform encoded by the RNA molecule.

The sequencing output from the methods defined herein may be used to identify the transcription start site, end site and/or splicing. The methods defined herein may comprise identifying at least one transcription start site, end site and/or splice junction after sequencing the processed RNA or cDNA (i.e. in the sequencing output), optionally identifying the transcription start site, end site and all splice junctions in one or more (2, 3, 4, 5, 10, 20, 100 or 1000 or more) transcript(s). These features typically represent a unique combination used by different cell types enabling the cell type to be identified. This information can also be used to identify the protein isoform that would be translated from an RNA sequence/molecule/transcript. Thus, the sequencing output may be used to identify the presence of an RNA transcript sequence. The sequencing output may be used to identify one or more isoforms of a protein. The protein may have 2, 3, 4, 5, 10, 15 or 20 or more isoforms and the method may identify which of the protein isoforms is encoded by the RNA molecule.

The RNA (transcript) sequence/molecule or set of RNA sequences may be associated with a disease or condition. An RNA (transcript) sequence/molecule or set of RNA sequences associated with a disease or condition is an RNA (transcript) sequence/molecule or set of RNA sequences that is correlated with the disease or condition. An RNA (transcript) sequence/molecule or set of RNA sequences associated with a disease or condition may be (uniquely) present in a subject with the disease or condition, optionally absent in a subject without the disease or condition. An RNA (transcript) sequence/molecule or set of RNA sequences associated with a disease or condition may be (uniquely) absent in a subject with the disease or condition, optionally present in a subject without the disease or condition. The RNA (transcript) sequence may have a coding sequence that is disease (for example cancer) specific. An RNA (transcript) sequence may encode a protein isoform that is associated with the disease or condition. One or more peptide sequence(s) may be identified in the protein isoform that are present only in cells from subjects with the disease, optionally cancer cells. One or more peptide sequence(s) may be identified in the protein isoform that are antigenic peptides. These peptide sequences (antigenic peptides) are encoded by sections of the RNA (transcript) sequence.

The invention provides a method for determining a set of RNA sequences associated with a disease or condition, the method comprising:
(a) providing an RNA sample obtained from a subject;
(b) processing the RNA sample, optionally comprising synthesizing cDNA using the RNA as a template;
(c) sequencing the processed RNA or cDNA, wherein the sequencing output is used to identify the presence of an RNA transcript sequence associated with a disease or condition; and
(d) identifying a set of RNA sequences in the RNA transcript.

The RNA sample may be extracted from any material, biological fluid, tissue, or cell obtained or otherwise derived from the subject. The sample may be a (solid) biopsy sample. The sample may be a liquid biopsy sample. The sample may be obtained from a biological fluid or a fluid or lysate generated from a biological material. The RNA sample may be extracted from a blood sample obtained from the subject.

The invention provides a method for determining a set of RNA sequences associated with a disease or condition, the method comprising:
(a) providing an RNA sample extracted from a blood sample obtained from a subject;
(b) processing the RNA sample, optionally comprising synthesizing cDNA using the RNA as a template;
(c) sequencing the processed RNA or cDNA, wherein the sequencing output is used to identify the presence of an RNA transcript sequence associated with a disease or condition; and
(d) identifying a set of RNA sequences in the RNA transcript.

The subject may have the disease or condition or the subject may be pregnant and the foetus may have the disease or condition.

In the methods, each RNA sequence in the set may encode an antigenic peptide. Each RNA sequence in the set may be (uniquely) expressed in a subject with the disease or condition and, optionally, is not expressed in subjects without the disease or condition. By "set of RNA sequences" is meant 2 or more, optionally 3, 4, 5, 6, 7, 8, 9, 10, 20, 50 or 100 or more RNA sequences. Each RNA sequence in the set may be more than 10 bp, 20 bp, 50 bp, 100 bp, 500 bp, or 1000 bp long, optionally 10 to 10000 bp, 20 to 1000 bp or 50 to 500 bp long. Each RNA sequence in the set may be less than 10 bp, 20 bp, 50 bp, 100 bp, 500 bp, or 1000 bp long. One or more, optionally 2, 3, 4, 5, 6, 7, 8, 9, 10, 20, 50 or 100 or more RNA sequences from the set may be used to produce a (first) RNA vaccine for the subject.

The invention provides a method for producing an RNA vaccine for a subject with a disease, the method comprising:
(i) providing an RNA sample obtained from the subject;
(ii) processing the RNA sample, optionally comprising synthesizing cDNA using the RNA as a template;
(iii) sequencing the processed RNA or cDNA, wherein the sequencing output is used to identify the presence of an RNA (transcript) sequence associated with the disease; and
(iv) using the identified RNA (transcript) sequence to produce a first RNA vaccine for the subject.

The RNA sample may be extracted from any material, biological fluid, tissue, or cell obtained or otherwise derived from the subject. The sample may be a (solid) biopsy sample. The sample may be a liquid biopsy sample. The sample may be obtained from a biological fluid or a fluid or lysate generated from a biological material. The RNA sample may be extracted from a blood sample obtained from the subject.

The invention provides a method for producing an RNA vaccine for a subject with a disease, the method comprising:
(i) providing an RNA sample extracted from a blood sample obtained from the subject;
(ii) processing the RNA sample, optionally comprising synthesizing cDNA using the RNA as a template;
(iii) sequencing the processed RNA or cDNA, wherein the sequencing output is used to identify the presence of an RNA (transcript) sequence associated with the disease; and
(iv) using the identified RNA (transcript) sequence to produce a first RNA vaccine for the subject.

The use of certain sample types such as blood samples is advantageous for continuous monitoring of subjects. Therefore, the method may further comprise:
(v) providing a further RNA sample (extracted from a blood sample) obtained from the subject at a later time point;
(vi) processing the RNA sample, optionally comprising synthesizing cDNA using the RNA as a template;
(vii) sequencing the processed RNA or cDNA, wherein the sequencing output is used to identify the presence of an RNA (transcript) sequence associated with the disease, and
(viii) using the identified RNA (transcript) sequence to produce a further RNA vaccine for the subject.

The first RNA vaccine and the further RNA vaccine may be the same. The first RNA vaccine and the further RNA vaccine may be different, optionally the first RNA vaccine and the further RNA vaccine differ in the antigenic peptides they encode. The RNA (transcript) sequence associated with the disease may be different in the RNA sample and the further RNA sample. The RNA (transcript) sequence associated with the disease may be the same in the RNA sample and the further RNA sample.

The RNA transcript may encode a protein isoform present in subjects with the disease. In the methods, using the identified RNA transcript sequence to produce the RNA vaccine may comprise producing an RNA molecule comprising at least a portion of the sequence, wherein the RNA molecule comprises an open reading frame (ORF), optionally encoding at least one antigenic peptide. The RNA molecule may further comprise a 5' UTR, 3' UTR, a polyA tail and/or a 5' cap. The 5' cap may have the Cap O structure or the Cap 1 structure. The Cap 0 structure may include a methyl-7 guanine nucleotide linked to the 5' position through a 5' triphosphate. The Cap 1 structure may be achieved by the methylation of the mRNA first nucleotide at the ribose 2'-O position. The RNA molecule may comprise one or two (optionally uridine-based) RNA strands, optionally with non-coding sequences optimised for translational performance. Thus, the RNA molecule in the RNA vaccine may comprise at least a portion of the wild-type sequence of the RNA transcript or may comprise a modified sequence. For example, the sequence may be adapted with respect to its codon usage. Adaption of codon usage can increase translation efficacy and half-life of the RNA. In the methods, at least 25%, preferably at least 30%, 40%, 50%, 60%, 70%, 80%, 90%, 95% or even 100% of uridine present in the RNA sequence of each RNA molecule in the RNA vaccine may be replaced by pseudouridine or N1-methylpseudouridine or 15-methyluridine or 2-thiouridine.

The RNA vaccine may comprise conventional (non-replicating) mRNA, self-amplifying mRNA and/or trans-amplifying mRNA (taRNA). Self-amplifying mRNA (saRNA) may be based on the addition of a viral replicase gene to enable the mRNA to self-replicate.

As used herein, the term "RNA vaccine" refers to a vaccine comprising an RNA molecule as defined herein. The vaccine may comprise, however, other substances and molecules which are required or which are advantageous when the vaccine is administered to an individual (e.g. pharmaceutical excipients). The RNA vaccine may comprise the RNA molecule in a buffer solution. The RNA molecule may be formulated in a lipid-based carrier, polymer or peptide, optionally a lipid nanoparticle. The RNA molecule may be formulated in a lipoplex nanoparticle comprising the synthetic cationic lipid (*R*)-*N*,*N*,*N*-trimethyl-2,3-dioleyloxy-1-propanaminium chloride (DOTMA) and the phospholipid 1,2-dioleoyl-sn-glycero-3-phosphatidylethanolamine (DOPE). The RNA molecule may be formulated to enable intravenous delivery. The RNA vaccine may be based on uridine mRNA-lipoplex nanoparticles (as described in Luis Rojas et al., Nature 2023; Jun 618(7963): 144-150 doi: 10.1038/s41586-023-06063-y, which is hereby incorporated by reference). The RNA vaccine may be delivered via transfection of dendritic cells. The RNA vaccine may encode more than 1, 2, 3, 4, 5, 6, 7, 8, 9, 10, 15 or 20 antigenic peptides.

The RNA vaccine may be manufactured as described in Sara Sousa Rosa et al. Vaccine. 2021 Apr 15;39(16):2190-2200 doi: 10.1016/j.vaccine.2021.03.038, which is hereby incorporated by reference.

In the methods, the disease may be cancer or an infectious disease (e.g. COVID-19). The RNA vaccine may be an RNA cancer vaccine. The RNA transcript sequence may have a coding region that is cancer specific. The RNA transcript sequence may encode a protein isoform that is (uniquely) expressed in subjects with cancer. The set of RNA sequences in the transcript may encode neoantigens (cancer specific peptide sub-sequences). The neoantigens may be expressed on the cell surface via MHC complexes. Neoantigens may be produced by alternative splicing and/or RNA editing and may be predicted from RNA sequencing output (Jiyeon Park and Yeun-Jun Chung, Genomics and Informatics 2019;17(3):e23 DOl: https://doi.org/10.5808/GI.2019.17.3.e23 which is hereby incorporated by reference). The neoantigens may be targets for therapy (for example immunotherapy), optionally a cancer vaccine, adoptive cell therapy and/or antibody-based therapy (Na Xie et al., Signal Transduction and Targeted Therapy (2023)8:9 https://doi.org/10.1038/s41392-022-01270-x, which is hereby incorporated by reference). 2 or more, 5 or more, 10 or more or 15 or more neoantigens may be targeted by an RNA vaccine.

The invention provides an RNA vaccine for use in therapy, wherein the RNA vaccine is produced using the methods defined herein. A subject may receive an immune checkpoint inhibitor, optionally atezolizumab, prior to treatment with the RNA vaccine. The RNA vaccine may be administered to the subject 1, 2, 3, 4, 5, 6, 7, 8, or 9 or more times.

The invention provides a method for discovering a biomarker for a disease comprising:
(i) providing an RNA sample obtained from a subject with the disease;
(ii) processing the RNA sample, optionally comprising synthesizing cDNA using the RNA as a template;
(iii) sequencing the processed RNA or cDNA; and
(iv) using the sequencing output to discover a disease biomarker.

The RNA sample may be extracted from a blood sample.

The invention provides a method for discovering a biomarker for a disease comprising:
(i) providing a first RNA sample and a second RNA sample;
(ii) processing the first and the second RNA samples, optionally comprising synthesizing cDNA using the RNA as a template;
(iii) sequencing the processed RNA or cDNA; and
(iv) comparing the sequencing output for the first and second samples to discover a disease biomarker.

The first RNA sample and second RNA sample may each be extracted from a blood sample.

The disease may be an autoimmune disease. Thus, there is also provided a method for discovering a biomarker for an autoimmune disease comprising:
(i) providing a first RNA sample extracted from a blood sample obtained from a subject with the disease and a second RNA sample extracted from a blood sample from a subject without the disease;
(ii) processing the first and the second RNA samples, optionally comprising synthesizing cDNA using the RNA as a template;
(iii) sequencing the processed RNA or cDNA; and
(iv) comparing the sequencing output for the first and second samples to discover a disease biomarker.

The invention provides a method for discovering a disease biomarker comprising:
(i) providing a first cDNA sample and a second cDNA sample;
(ii) normalizing the first and the second cDNA samples;
(iii) sequencing the normalized first and second cDNA samples; and
(iv) comparing the sequencing output for the first and second cDNA samples to discover a disease biomarker.

The first RNA/cDNA sample and the second RNA/cDNA sample may be from the same subject. The first RNA/cDNA sample may be from the subject prior to treatment for a disease and the second RNA/cDNA sample may be from the same subject after treatment for the disease. The first RNA/cDNA sample may be from the subject prior to treatment for a disease and the second RNA/cDNA sample may be from the same subject after treatment for the disease has started and/or after treatment for the disease has been completed.

The first RNA/cDNA sample and the second RNA/cDNA sample may be from different subjects. In the methods, the first RNA/cDNA sample and the second RNA/cDNA sample may be from subjects with the same disease at different grades or stages. The first RNA/cDNA sample and the second RNA/cDNA sample may be from subjects with different diseases, for example different types of cancer. The first RNA/cDNA sample may be from a subject with a disease and the second RNA/cDNA sample is from a subject without the disease.

The invention provides a method for discovering a disease biomarker comprising:
(i) providing a first cDNA sample from a subject with a disease and a second cDNA sample from a subject without the disease;
(ii) normalizing the first and the second cDNA samples;
(iii) sequencing the normalized first and second cDNA samples; and
(iv) comparing the sequencing output for the first and second cDNA samples to discover a disease biomarker.

Discovering a disease biomarker means identifying a novel biomarker (an indicator of a biological state) for a particular disease, for example uncovering a previously unknown biomarker for developing into a test for the disease. The disease biomarker may be suitable for use in diagnosing the disease, characterising the disease, predicting response to therapy, detecting minimal residual disease and/or prognosing the disease. By characterisation is meant classification and evaluation of the disease. Prognosis refers to predicting the likely outcome of the disease for the subject. The characterisation of and/or prognosis for the disease may comprise determining the grade and/or stage of the disease. The characterisation of the disease may comprise determining the sub-type of the disease. The disease biomarker may be suitable for use in indicating the likelihood that a subject with a particular disease will benefit from a specific therapy.

In the methods, the disease may be cancer. The characterisation of and/or prognosis for the cancer may comprise determining the presence or absence of metastases. Metastasis, or metastatic disease, is the spread of a cancer from one organ or part to another non-adjacent organ or part. The new occurrences of disease thus generated are referred to as metastases. Characterisation of and/or prognosis for the disease may also comprise predicting biochemical recurrence and/or determining whether the cancer is aggressive and/or determining whether the cancer has spread to the lymph nodes. Aggressive refers to a cancer that is fast growing, more likely to spread, more likely to recur and/or shows resistance to treatment.

The invention provides a method for monitoring a subject comprising:
(i) providing a first RNA sample extracted from a blood sample obtained from the subject at a first time point and a second RNA sample extracted from a blood sample from the subject at a second time point;
(ii) processing the first and the second RNA samples, optionally comprising synthesizing cDNA using the RNA as a template;
(iii) sequencing the processed RNA or cDNA; and
(iv) comparing the sequencing output for the first and second samples.

There is also provided a method for monitoring a subject comprising:
(i) providing a first cDNA sample from the subject at a first time point and a second cDNA sample from the subject at a second time point;
(ii) normalizing the first and the second cDNA samples;
(iii) sequencing the normalized first and second cDNA samples; and
(iv) comparing the sequencing output for the first and second cDNA samples.

Monitoring a subject may comprise monitoring response to treatment for a disease, for example monitoring whether treatment is successful and/or monitoring for adverse reactions/complications. The first time point may be prior to starting treatment and the second time point may be during or after treatment. Comparing the sequencing output for the first and second RNA/cDNA samples may provide an indication as to whether treatment has been successful. For example, the presence or absence of a disease biomarker may indicate whether treatment has been successful. Comparing the sequencing output for the first and second RNA/cDNA samples may comprise comparing to each other and/or to the sequencing output from a reference sample.

In the methods, an immune system related transcript may be detected in the sequencing output. The first time point may be prior to starting treatment with an immunotherapy and the second time point may be during or after treatment with the immunotherapy.

In the methods, the disease biomarker may be a cDNA sequence or an RNA sequence. The cDNA sequence will correspond to an RNA sequence. The cDNA/RNA sequence may correspond to a protein or peptide. The method may further comprise identifying an RNA, transcript, transcript model, gene, protein and/or peptide corresponding to a cDNA sequence. The disease biomarker may, therefore, be a cDNA molecule (of a specific sequence), DNA molecule (of a specific sequence), RNA molecule (of a specific sequence), transcript, transcript model, protein or peptide.

The method may comprise discovering more than one disease biomarker, optionally more than 10, 100, 1000, 10000, 100000, 1 million or 10 million disease biomarkers. The method may comprise discovering between 1 and 10, 1 and 100, 1 and 1000, 1 and 10000, 1 and 100000, 1 and 1 million or 1 and 10 million disease biomarkers.

Two or more of the disease biomarkers may be compiled to form a database. The invention provides a method for producing a database of disease biomarkers, the method comprising:
(i) providing two or more RNA samples obtained from one or more subjects with a disease;
(ii) processing the RNA samples, optionally comprising synthesizing cDNA using the RNA as a template;
(iii) sequencing the processed RNA or cDNA, wherein the sequencing output is used to identify two or more disease biomarkers; and
(iv) compiling the disease biomarkers to form a database.

The RNA samples may each be extracted from a blood sample. The invention provides a method for producing a database of disease biomarkers, the method comprising:
(i) providing two or more RNA samples extracted from blood samples obtained from one or more subjects with a disease;
(ii) processing the RNA samples, optionally comprising synthesizing cDNA using the RNA as a template;
(iii) sequencing the processed RNA or cDNA, wherein the sequencing output is used to identify two or more disease biomarkers; and
(iv) compiling the disease biomarkers to form a database.

Each disease biomarker may be an RNA transcript, optionally wherein the RNA transcript encodes a protein isoform. The protein isoform may be present or absent in subjects with the disease.

The invention provides a method for producing a database of RNA sequences associated with a disease or condition, the method comprising:
(i) providing two or more RNA samples obtained from one or more subjects;
(ii) processing the RNA samples, optionally comprising synthesizing cDNA using the RNA as a template;
(iii) sequencing the processed RNA or cDNA, wherein the sequencing output is used to identify the presence of two or more RNA transcript sequences associated with a disease or condition;
(iv) identifying a set of RNA sequences in each RNA transcript; and
(v) compiling the sets of RNA sequences to form a database.

The RNA samples may each be extracted from a blood sample. The invention provides a method for producing a database of RNA sequences associated with a disease or condition, the method comprising:
(i) providing two or more RNA samples extracted from blood samples obtained from one or more subjects;
(ii) processing the RNA samples, optionally comprising synthesizing cDNA using the RNA as a template;
(iii) sequencing the processed RNA or cDNA, wherein the sequencing output is used to identify the presence of two or more RNA transcript sequences associated with a disease or condition;
(iv) identifying a set of RNA sequences in each RNA transcript; and
(v) compiling the sets of RNA sequences to form a database.

In the methods, each RNA sequence in the set may encode an antigenic peptide.

The sequencing output from a sample analysed and/or processed according to the methods defined herein may be compared to a database produced using a method defined herein in order to identify the presence of a disease biomarker or a set of RNA sequences associated with a disease or condition.

The database of disease biomarkers and the database of RNA sequences associated with a disease or condition may be used to guide treatment decisions and to aid in the development of new treatments. The disease biomarker and/or set of RNA sequences may be a suitable target for a therapeutic agent, for example a vaccine, an RNA therapy and/or gene editing. The discovery of a disease biomarker specific to cancer cells can be an initial step in identifying a cancer specific antigen for a cancer vaccine to target. Thus, the method may further comprise identifying a transcript or protein corresponding to the disease biomarker as a target for therapy, optionally a cancer vaccine target. The method may further comprise developing a cancer vaccine, optionally an RNA vaccine, directed to the target.

The invention provides a method for discovering a cancer vaccine target comprising:
(i) providing a first RNA/cDNA sample from a subject with a cancer and a second RNA/cDNA sample from a subject without the cancer;
(ii) normalizing the first and the second RNA/cDNA samples;
(iii) sequencing the normalized first and second RNA/cDNA samples; and
(iv) comparing the sequencing output for the first and second RNA/cDNA samples to discover a cancer vaccine target.

The methods may comprise providing 2, 3, 4, 5, 6, 7, 8, 9, 10, 20, 30, 40 or more RNA/cDNA samples from different subjects with the disease and/or providing 2, 3, 4, 5, 6, 7, 8, 9, 10, 20, 30, 40 or more RNA/cDNA samples from different subjects without the disease. Preferably, the methods comprise providing 30 or more RNA/cDNA samples from different subjects with the disease (e.g. with a cancer) and providing 30 or more RNA/cDNA samples from different subjects without the disease (e.g. without the cancer).

In the methods, the first RNA/cDNA sample and the second RNA/cDNA sample may be obtained from biological fluid or a fluid or lysate generated from a biological material. The first RNA/cDNA sample and the second RNA/cDNA sample may be obtained from blood. The first RNA/cDNA sample may be obtained by extracting RNA from a biological sample (e.g. blood) obtained from the subject with the disease and the second RNA/cDNA sample may be obtained by extracting RNA from a biological sample (e.g. blood) obtained from the subject without the disease. Optionally cDNA is then synthesized using the RNA as a template (i.e. by reverse transcription). Thus, the methods may further comprise:
(a) extracting RNA from biological fluid (e.g. blood) or a fluid or lysate generated from a biological material from a subject with a disease and from a subject without the disease; and
(b) synthesizing cDNA using the RNA as a template (i.e. converting the RNA into cDNA).
In this way the first cDNA sample and the second cDNA sample may be produced.

A subject with a disease means the subject has the disease at the time the (biological) sample (biological fluid or biological material) from which the RNA/cDNA sample is derived is taken from the subject. A subject without a disease means the subject does not have the disease at the time the (biological) sample (biological fluid or biological material) from which the RNA/cDNA sample is derived is taken from the subject.

The subject without the disease may be a healthy subject.

The disease biomarker may be a cDNA molecule (of a specific sequence), RNA molecule (of a specific sequence), protein or peptide that is detectable in a sample from a subject with a disease but not in a sample from a subject without the disease. Alternatively, the disease biomarker may be a cDNA molecule (of a specific sequence), RNA molecule (of a specific sequence), protein or peptide that is not detectable in a sample from a subject with a disease but is detectable in a sample from a subject without the disease.

The cancer vaccine target may be a cDNA molecule (of a specific sequence), RNA molecule (of a specific sequence), protein or peptide that is detectable in a sample from a subject with a cancer but not in a sample from a subject without the cancer.

The disease biomarker may be a RNA/cDNA sequence that is present in the first RNA/cDNA sample but not in the second RNA/cDNA sample or is present in the second RNA/cDNA sample but not in the first RNA/cDNA sample. The disease biomarker may be a transcript that is (uniquely) present in subjects with a particular disease. The disease biomarker may be a transcript that is (uniquely) absent in subjects with a particular disease.

The cancer vaccine target may be a RNA/cDNA sequence that is present in the first RNA/cDNA sample but not in the second RNA/cDNA sample. The cancer vaccine target may be a transcript that is uniquely present in subjects with a particular cancer. The transcript/RNA/cDNA sequence may correspond to a particular protein or peptide. At least a portion of the protein or peptide may form an antigen comprised in a cancer vaccine.

The disease biomarker may be a transcript that is (uniquely) present in subjects with a particular disease. The disease biomarker may be a transcript that is (uniquely) absent in subjects with a particular disease. The cancer vaccine target may be a transcript that is uniquely present in subjects with a particular cancer. The transcript/RNA/cDNA sequence may correspond to a particular protein or peptide. At least a portion of the protein or peptide may form an antigen comprised in a cancer vaccine.

The present invention enables the identification of transcripts found only in subjects with a disease, optionally cancer. Such transcript models can be identified through comparison with subjects without the disease (e.g. benign patients) and, optionally, public transcriptome annotation databases. Sequencing output and/or resulting transcriptomic profile(s) from a subject with a particular disease (e.g. breast cancer) can be compared with sequencing output and/or resulting transcriptomic profile(s) from a subject with a different disease (for example, ovarian and/or colorectal cancer) to determine if the biomarker is unique to the particular disease (e.g. breast cancer).

The invention provides a method for diagnosing a disease in a subject comprising:
(i) providing an RNA sample (extracted from a blood sample) obtained from the subject;
(ii) processing the RNA sample, optionally comprising synthesizing cDNA using the RNA as a template; and
(iii) sequencing the processed RNA or cDNA, wherein the sequencing output is used to identify whether the subject has the disease.

The disease may be an autoimmune disease, a cancer, diabetes, coronary disease, a metabolic disease, Alzheimer's disease, dementia, and/or an infectious disease. The disease may be a viral infection, bacterial infection and/or fungal infection. The disease may be COVID-19. The disease may be identified at a (very) early stage, optionally before symptoms have developed. The disease may be cancer and the cancer may be identified at a (very) early stage, optionally before significant tumour growth has occurred.

The disease may be cancer (e.g. a hematological cancer such as leukemia, lymphoma or multiple myeloma) and diagnosing the disease may comprise detecting minimal residual disease (MRD). MRD may be defined as cancer cells that remain in the subject during or after treatment.

The disease may be an autoimmune disease.

The invention provides a method for diagnosing an autoimmune disease in a subject comprising:
(i) providing an RNA sample obtained from the subject;
(ii) processing the RNA sample, optionally comprising synthesizing cDNA using the RNA as a template; and
(iii) sequencing the processed RNA or cDNA, wherein the sequencing output is used to identify whether the subject has the disease.

The RNA sample may be extracted from a blood sample.

The invention provides a method for diagnosing an autoimmune disease in a subject comprising:
(i) providing an RNA sample extracted from a blood sample obtained from the subject;
(ii) processing the RNA sample, optionally comprising synthesizing cDNA using the RNA as a template; and
(iii) sequencing the processed RNA or cDNA, wherein the sequencing output is used to identify whether the subject has the disease.

An "autoimmune disease" herein is a disease or disorder wherein the immune system of a subject mounts an immune response to the subject's own tissue. The autoimmune disease may be arthritis, celiac disease, diabetes mellitus type 1, graves' disease, inflammatory bowel disease, multiple sclerosis, alopecia areata, Addison's disease, pernicious anemia, psoriasis, systemic lupus erythematosus, myasthenia gravis, Hashimoto's thyroiditis, Vitiligo, Sjogren's syndrome, myositis, chronic inflammatory demyelinating polyneuropathy (CIDP), dermatomyositis, Guillain-Barre syndrome, ulcerative colitis, Crohn's disease and/or vasculitis.

The invention provides a method for diagnosing a disease in a subject comprising:
(i) providing a RNA/cDNA sample from the subject;
(ii) normalizing the RNA/cDNA sample; and
(iii) sequencing the normalized RNA/cDNA sample, wherein the sequencing output is used to identify whether the subject has the disease.

The sequencing output may be used to identify whether the subject has the disease by comparing to a database of biomarkers, optionally wherein the database of biomarkers is produced using a method defined herein.

By diagnosing is meant determining that a subject has the disease at the time of testing.

The methods defined herein may further comprise selecting a treatment appropriate for the disease and, optionally, treating the disease with the selected treatment. Treating the subject may start at an early stage of disease progression, optionally before symptoms have appeared or significant tumour growth has occurred.

The invention provides a method for characterising and/or prognosing a disease in a subject comprising:
(i) providing a RNA/cDNA sample from the subject;
(ii) processing (normalizing) the RNA/cDNA sample; and
(iii) sequencing the processed (normalized) RNA/cDNA sample, wherein the sequencing output is used to provide a characterisation of and/or a prognosis for the disease.

The invention provides a method for selecting a treatment for a disease in a subject comprising:
(i) providing a RNA/cDNA sample from the subject;
(ii) processing (normalizing) the RNA/cDNA sample;
(iii) sequencing the processed (normalized) RNA/cDNA sample, wherein the sequencing output is used to provide a diagnosis, characterisation of and/or a prognosis for the disease; and
(iv) selecting a treatment appropriate to the diagnosis, characterisation of and/or prognosis for the disease.

The invention provides a method for predicting the responsiveness of a subject with a disease to a therapeutic agent comprising:
(i) providing a RNA/cDNA sample from the subject;
(ii) processing (normalizing) the RNA/cDNA sample; and
(iii) sequencing the processed (normalized) RNA/cDNA sample, wherein the sequencing output is used to predict the responsiveness of the subject to the therapeutic agent.

The therapeutic agent may be an immune checkpoint inhibitor and/or immunotherapy, optionally CAR-T therapy. The RNA/cDNA sample may comprise full-length RNA/cDNA and/or the processed RNA/cDNA sample comprises full-length RNA/cDNA.

The methods as described herein may further comprise treating the subject.

The methods may comprise comparing the sequencing output for the processed (normalized) RNA/cDNA sample to one or more reference sequences or to the sequencing output of one or more control samples, optionally wherein the one or more control samples are from one or more subjects with and/or without the disease. Preferably, the methods comprise comparing the sequencing output for the processed (normalized) RNA/cDNA sample to the sequencing output of one or more control samples from one or more subjects with the disease.

By sequencing output is meant one or more sequences obtained from sequencing the processed (normalized) RNA/cDNA. The sequence(s) may be raw sequence(s) or may be further processed. For example, low quality reads may be filtered and/or adapter sequences may be filtered and removed. The (processed) sequence(s) may be mapped to the human reference genome (for example, using Minimap2) to prepare transcriptome profile(s). One or more transcript models may be identified in the transcriptome profile(s) (sequence(s) mapped to the genome). The transcript model represents a specific transcript i.e. a particular RNA isoform or splice variant produced from a gene. The sequencing output that is used in the methods defined herein (for example, that is compared to discover a disease biomarker or is used to identify whether the subject has a disease) may be transcript(s), transcriptome profile(s) and/or transcript model(s).

Using the sequencing output to identify whether the subject has the disease may comprise detecting a disease biomarker. Using the sequencing output to identify whether the subject has the disease may comprise detecting more than one disease biomarker, optionally more than 10, 100, 1000, 10000, 100000, 1 million or 10 million disease biomarkers. Using the sequencing output to identify whether the subject has the disease may comprise detecting between 1 and 10, 1 and 100, 1 and 1000, 1 and 10000, 1 and 100000, 1 and 1 million or 1 and 10 million disease biomarkers. Detecting the disease biomarker may comprise determining the presence or absence of the disease biomarker. Using the sequencing output to identify whether the subject has the disease may comprise determining the presence or absence of more than one disease biomarker, optionally more than 10, 100, 1000, 10000, 100000, 1 million or 10 million disease biomarkers. Using the sequencing output to identify whether the subject has the disease may comprise determining the presence or absence of between 1 and 10, 1 and 100, 1 and 1000, 1 and 10000, 1 and 100000, 1 and 1 million or 1 and 10 million disease biomarkers.

The presence of a particular RNA/cDNA sequence in the sequencing output may indicate that the subject has the disease, for example where a particular transcript (corresponding to the cDNA molecule) is uniquely present in subjects with a particular disease. Likewise, the presence of a particular RNA/cDNA sequence in the sequencing output may indicate a characterisation of and/or a prognosis for the disease. The presence of a particular RNA/cDNA sequence in the sequencing output may allow prediction of the responsiveness of a subject with a disease to a therapeutic agent, for example where a particular transcript has been found to correlate with responsiveness of a subject with a disease to a particular therapeutic agent.

The absence of a particular RNA/cDNA sequence in the sequencing output may indicate that the subject has the disease, for example where a particular transcript (corresponding to the cDNA molecule) is absent in subjects with a particular disease. Likewise, the absence of a particular RNA/cDNA sequence in the sequencing output may indicate a characterisation of and/or a prognosis for the disease. The absence of a particular RNA/cDNA sequence in the sequencing output may allow prediction of the responsiveness of a subject with a disease to a therapeutic agent, for example where a particular transcript has been found to correlate with responsiveness of a subject with a disease to a particular therapeutic agent.

RNA samples may be obtained from biological samples of any suitable form including any material, biological fluid, tissue, or cell obtained or otherwise derived from a subject. The sample may include cancer cells or genetic material (DNA or RNA) derived from the cancer cells, to include cell-free genetic material (e.g. found in the peripheral blood). The sample may comprise a biopsy sample (e.g. a formalin-fixed paraffin-embedded biopsy sample). The sample may comprise a fresh/frozen (FF) sample. The sample may comprise tumour (cancer) tissue, optionally breast tumour (cancer) tissue. The sample may comprise tumour (cancer) cells, optionally breast tumour (cancer) cells. The tissue sample may be obtained by any suitable technique. Examples include a biopsy procedure, optionally a fine needle aspirate biopsy procedure. Body fluid samples may also be utilised. Suitable sample types include blood (including whole blood, leukocytes, peripheral blood mononuclear cells, buffy coat, plasma, and serum), sputum, tears, mucus, nasal washes, nasal aspirate, breath, urine, semen, saliva, meningeal fluid, amniotic fluid, glandular fluid, lymph fluid, nipple aspirate, bronchial aspirate, synovial fluid, joint aspirate, ascites, cells, a cellular extract, and cerebrospinal fluid. This also includes experimentally separated fractions of all of the preceding. For example, a blood sample can be fractionated into serum or into fractions containing particular types of blood cells, such as red blood cells or white blood cells (leukocytes). If desired, a sample can be a combination of samples from a subject, such as a combination of a tissue and fluid sample. The term "sample" also includes materials containing homogenized solid material, such as from a stool sample, a tissue sample, or a tissue biopsy, for example. The term "sample" also includes materials derived from a tissue culture or a cell culture, including tissue resection and biopsy samples. Example methods for obtaining a sample include, e.g., phlebotomy, swab (e.g., buccal swab). Samples can also be collected, e.g., by micro dissection (e.g., laser capture micro dissection (LCM) or laser micro dissection (LMD)), bladder wash, smear (e.g., a PAP smear), or ductal lavage. A "sample" obtained or derived from a subject includes any such sample that has been processed in any suitable manner after being obtained from the subject. The methods of the invention as defined herein may begin with an obtained sample and thus do not necessarily incorporate the step of obtaining the sample from the patient.

In the methods, the RNA/cDNA sample may be obtained from a tumour (e.g. a solid biopsy) or from biological fluid or a fluid or lysate generated from a biological material. The RNA/cDNA sample may be obtained from blood. The RNA/cDNA sample may be obtained by extracting RNA from a biological sample (e.g. blood) obtained from the subject. cDNA is then synthesized using the RNA as a template (i.e. by reverse transcription). Thus, the method may further comprise:
(a) extracting RNA from biological fluid (e.g. blood) or a fluid or lysate generated from a biological material from the subject; and/or
(b) synthesizing cDNA using the RNA as a template (i.e. converting the RNA into cDNA).
In this way the cDNA sample from the subject may be produced.

The methods may further comprise reporting to the subject the outcome of the method. The result may be a diagnosis or prognosis for the disease. The result may be a specific grade or stage of a disease, such as a cancer.

The term "sequence" may refer to all of the individual nucleic acid (e.g. cDNA or RNA) molecules having a 100% identical nucleotide sequence. Alternatively, the term "sequence" may refer to all of the individual nucleic acid (e.g. cDNA or RNA) molecules having more than 99%, 98%, 97%, 96%, 95%, 90%, 85%, 80%, 75%, 70%, 65%, 60%, 55% or 50% identity to one another. "% identity" between a query nucleic acid sequence and a subject nucleic acid sequence may be calculated using a suitable algorithm (e.g. BLASTN, FASTA, Needleman-Wunsch, Smith-Waterman, LALIGN, or GenePAST/KERR) or software (e.g. DNASTAR Lasergene, GenomeQuest, EMBOSS needle or EMBOSS infoalign), over the entire length of the query sequence after a pair-wise global sequence alignment has been performed using a suitable algorithm (e.g. Needleman-Wunsch or GenePAST/KERR) or software (e.g. DNASTAR Lasergene or GenePAST/KERR). The term "unique sequence" or "unique cDNA sequence" or "unique RNA sequence" may refer to all of the individual nucleic acid (e.g. cDNA or RNA as appropriate) molecules which meet or exceed a threshold % identity (e.g. 100%, 99%, 98%, 97%, 96%, 95%, 90%, 85%, 80%, 75%, 70%, 65%, 60%, 55% or 50% identity to one another). The "unique sequence" or "unique cDNA sequence" or "unique RNA sequence" may differ from the other sequences present in the sample (for example, by at least 1, 2, 3, 4, 5, 6, 7, 8, 9, 10, 20, 50 or 100 nucleotides or by at least 1%, 2%, 3%, 4%, 5%, 10%, 15%, 20%, 25%, 30%, 35%, 40%, 45% or 50% of their sequence).

In the methods, the disease may not be an infectious disease. The disease may be cancer. The cancer may be an epithelial cancer. The cancer may be breast, ovarian and/or colorectal cancer. Preferably, the cancer is breast cancer. The first RNA/cDNA sample may be from a subject with breast cancer and the second RNA/cDNA sample may be from a subject with a benign breast condition.

The method may be used to diagnose more than one disease in a single process, for example through detection of multiple RNA or cDNA molecules (derived from transcripts) that are each uniquely present in subjects with a particular disease. The method may be used to diagnose more than one autoimmune disease.

The method may be used to diagnose more than one cancer type. The method may be used to distinguish between breast cancer and a benign breast condition.

In the methods, processing the RNA sample may comprise normalization (reducing the variability in the levels of different RNA or cDNA sequences in the sample). Thus, the processed RNA or cDNA sample may be a normalized RNA or cDNA sample.

Processing the RNA sample may comprise equalizing the sample. Thus, in the processed RNA or cDNA the relative abundance of all the unique RNA or cDNA sequences may be more equal. For example, the levels of the unique sequences in the processed RNA or cDNA sample may vary by less than 50%, less than 40%, less than 30%, less than 20%, or less than 10%.

In the methods, processing an RNA or cDNA sample may reduce the variability in the levels of the RNA or cDNA (e.g. by at least 10%, at least 20%, at least 30%, at least 40%, at least 50%, at least 60%, at least 70%, at least 80%, or at least 90%). Processing RNA or cDNA may achieve a more uniform distribution of cDNA sequences. The difference in abundance between the most abundant RNA/cDNA and the least abundant RNA/cDNA in the sample may be reduced (e.g. by at least 10%, at least 20%, at least 30%, at least 40%, at least 50%, at least 60%, at least 70%, at least 80%, or at least 90%). In the methods, processing the RNA or cDNA sample may reduce the number of molecules (copy number) of the (1, 10, 100, 1000, or 10000) most abundant RNA or cDNA molecule(s) by at least 10%, at least 20%, at least 30%, at least 40%, at least 50%, at least 60%, at least 70%, at least 80%, or at least 90%. The number of molecules (copy number) of the most abundant RNA or cDNA molecule in the (first and/or second) RNA or cDNA sample may be reduced by at least 50% in the processed RNA or cDNA. The relative abundance of the (1, 10, 100, 1000, or 10000) least abundant RNA or cDNA molecule(s) may be increased by at least 10%, at least 20%, at least 30%, at least 40%, at least 50%, at least 60%, at least 70%, at least 80%, or at least 90%. The number of molecules (copy number) of the least abundant RNA or cDNA molecule in the (first and/or second) cDNA sample may be increased by at least 50% in the processed RNA or cDNA.

The processed RNA or cDNA sample may be more readily analysable. It may be more efficiently sequenced because the relative representation or levels of less abundant sequences is increased.

Normalizing an RNA or cDNA sample results in production of a normalized RNA or cDNA sample. Normalizing may comprise (selectively) increasing the relative abundance of less abundant sequences without targeting specific sequences based on their nucleotide sequence (i.e. identity or homology to a known sequence).

By "normalized" is meant that the levels of RNA or cDNA sequences in the sample are more equal. Thus, a normalized RNA or cDNA sample may be one in which the amount of each unique RNA or cDNA sequence is more uniform than in the same sample prior to normalization i.e. a normalized RNA or cDNA sample is closer to achieving each unique RNA or cDNA sequence having the same abundance (relative to other unique RNA or cDNA sequences within the normalized RNA or cDNA sample) than the same sample prior to normalization. To achieve this the relative representation or levels of less abundant sequences may be increased and/or the relative representation or levels of more abundant sequences may be decreased. The increase in less abundant sequences/decrease in more abundant sequences is selective in the sense that if all sequences were increased/decreased to the same degree the relative abundance would stay the same. However, the relative representation or levels of less abundant sequences may be increased and/or the relative representation or levels of more abundant sequences may be decreased without targeting (for example, using pre-defined probes) specific sequences based on their nucleotide composition (i.e. based on their identity or homology to a known sequence). The less abundant sequences may be the unique sequences with an amount that is below a threshold, for example they are present in the RNA or cDNA sample prior to normalization in an amount that is below the mean amount for a unique sequence in the sample. The less abundant sequences may be present in the RNA or cDNA sample prior to normalization at an amount that is 0.1 %, 1%, 10%, 20%, 30%, 40%, 50%, 60%, 70, 80% or 90% below the mean amount for a unique sequence in the sample. The more abundant sequences may be the unique sequences with an amount that is above a threshold, for example they are present in the RNA or cDNA sample prior to normalization in an amount that is above the mean amount for a unique sequence in the sample. The more abundant sequences may be present in the RNA or cDNA sample prior to normalization at an amount that is 0.1 %, 1 %, 10%, 20%, 30%, 40%, 50%, 60%, 70, 80% or 90% above the mean amount for a unique sequence in the sample. By relative abundance is meant abundance relative to other unique sequences in the sample.

A normalized RNA or cDNA sample may comprise RNA or cDNA sequences having substantially the same levels. For example, wherein the levels of the sequences of the normalized RNA or cDNA vary by less than 50%, less than 40%, less than 30%, less than 20%, or less than 10%. The normalized RNA or cDNA may be a normalized RNA or cDNA sample in which at least a portion of the 10, 100, 1000, or 10000 most abundant (unique) sequences in the RNA or cDNA sample have been removed or reduced (by at least 1%, 10%, 20%, 30%, 40%, 50%, 60%, 70%, 80% or 90%) in copy number. The normalized RNA or cDNA may be a normalized RNA or cDNA sample in which levels of at least a portion of the 10, 100, 1000, or 10000 least abundant (unique) sequences in the RNA or cDNA sample have been increased e.g. by at least 1%, 10%, 20%, 30%, 40%, 50%, 60%, 70%, 80% or 90% in copy number. The methods for normalizing RNA or cDNA sample(s) described herein may be methods for equalizing cDNA sample(s) i.e. equalizing the relative abundances of each unique sequence.

In the methods, normalizing the (first and/or the second) RNA or cDNA sample(s) may increase the amount (copy number) of at least a portion of the low abundance RNA or cDNA sequences within the (first and/or the second) cDNA sample(s). The low abundance RNA or cDNA sequences may be the 50%, 40%, 30%, 20%, 10% or 1% of (unique) sequences with the lowest copy number. Thus, normalizing may comprise selectively increasing the amount of low abundance RNA or cDNA within each RNA or cDNA sample.

In the methods, normalized RNA or cDNA may be RNA or cDNA that is more readily analysable. It may be more efficiently sequenced because the relative representation of less abundant sequences is increased. Normalizing the (first and/or the second) RNA or cDNA sample(s) may not comprise removing abundant (more abundant) RNA or cDNA molecules/sequences (such as those corresponding to Albumin, IgG, Apolipoprotein A-I, Transferrin, Apolipoprotein A-II, α₁-Proteinase inhibitor, α₁-Acid glycoprotein, Transthyretin, Hepatoglobin and/or Hemopexin) from the sample(s) (for example, using duplex-specific nuclease or sequence targeted methods). Normalizing may not comprise targeting specific (unique) sequences (such as those corresponding to Albumin, IgG, Apolipoprotein A-I, Transferrin, Apolipoprotein A-II, α₁-Proteinase inhibitor, α₁-Acid glycoprotein, Transthyretin, Hepatoglobin and/or Hemopexin). Normalizing a RNA or cDNA sample may be non-targeted i.e. it may not involve targeting specific sequences based on their nucleotide sequence (for example, it may not involve targeting a particular sequence based on its identity or homology to a known sequence).

Processing the RNA sample(s) may improve detection of low-abundance RNA or cDNA, optionally wherein processing the RNA sample(s) comprises increasing the amount of low abundance RNA or cDNA within each sample.

Methods that may be used for processing an RNA or cDNA sample include depletion methods (such as CRISPR-based depletion methods), methods that comprise inhibiting reverse transcription of abundant RNA sequences (e.g. inhibition of cDNA synthesis using oligo blockers) and normalization (e.g. cDNA normalization) methods.

Depletion methods comprise removing unwanted RNA or cDNA molecules/sequences. These may be contaminating RNA or cDNA molecules/sequences (for example bacterial transcripts, optionally bacterial ribosomal RNA) or abundant (more abundant) RNA or cDNA molecules/sequences (such as those corresponding to ribosomal, mitochondrial, globin and housekeeping genes, optionally those corresponding to Albumin, IgG, Apolipoprotein A-I, Transferrin, Apolipoprotein A-II, α₁-Proteinase inhibitor, α₁-Acid glycoprotein, Transthyretin, Hepatoglobin and/or Hemopexin) from the sample(s). CRISPR-Cas9 may be used to degrade abundant sequences. Optionally, CRISPR-Cas9 complexes are formed with a pool of designed guide RNAs, and the complexes are mixed with a cDNA sample. After the unwanted abundant sequences are cut, they cannot be substrates for PCR amplification and subsequent sequencing. Example products include CRISPclean^{™} Stranded Total RNA Prep with rRNA Depletion from Jumpcode Genomics.

Methods that comprise inhibition of reverse transcription may use high-affinity RNA-binding oligonucleotides to block reverse transcription and/or PCR amplification of specific RNA transcripts (see, for example, Everaert C et al., Biological Procedures Online 25, Article number: 7 (2023) https://doi.org/10.1186/s12575-023-00193-3, which is hereby incorporated by reference). An LNA-modified oligonucleotide complementary to an unwanted RNA can be designed, which can block reverse transcription and/or PCR amplification when bound downstream of the priming site.

Complementary DNA (cDNA) normalization (Alex S. Shcheglov, Pavel A. Zhulidov, Ekaterina A. Bogdanova, D. A. S. Normalization of cDNA Libraries, Nucleic Acids Hybrid. CHAPTER 5, (2014)) addresses issues with high abundance house-keeping genes reducing sampling efficiency for genes of interest. Since RNA sequencing typically relies on the conversion of RNA to double stranded cDNA, cDNA normalization takes advantage of the biochemical properties of cDNA to generate a uniform distribution of unique genes and isoforms within a cDNA library. In theory, the maximum non-targeted sampling efficiency is produced if all unique RNA sequences are represented at the same relative abundance. Thus, the objective of normalization is to re-distribute a cDNA library (sample) to meet this criterion as closely as possible.

Complementary DNA (cDNA) normalization may be full length cDNA normalization. Complementary DNA (cDNA) normalization may be performed by the Duplex Specific Nuclease (DSN) method (see e.g. Zhulidov, P. A. et al. Simple cDNA normalization using kamchatka crab duplex-specific nuclease. Nucleic Acids Res. 32, e37 (2004) which is hereby incorporated by reference) or the hydroxyapatite column method (see e.g. Andrews-Pfannkoch, C., Fadrosh, D. W., Thorpe, J. & Williamson, S. J. Hydroxyapatite-mediated separation of double-stranded DNA, single-stranded DNA, and RNA genomes from natural viral assemblages. Appl. Environ. Microbiol. 76, 5039-5045 (2010) which is hereby incorporated by reference). Both methods rely on the denaturation and re-hybridization of cDNA strands. As the single stranded cDNA move about in solution, the sequences that are more highly abundant have a greater probability of finding a matching complementary sequence with which to re-hybridize. Thus, as re-hybridization reaches its limit, the remaining single stranded cDNA represents a normalized sequence library.

Thus, processing the RNA sample may comprise synthesizing double stranded cDNA using the RNA as a template and then denaturing and re-hybridizing the cDNA strands.

The difference between the DSN method and the hydroxyapatite column method lies in their approach for isolating the single stranded cDNA library from the re-hybridized double stranded cDNA molecules.

In the DSN method, an enzyme which specifically cleaves double stranded DNA is used to decompose all double stranded cDNA within the solution. The solution is then purified and size-selected for cDNA sequences above a certain length. These sequences are then amplified using the Polymerase Chain Reaction (PCR).

In the column method, the denatured and re-hybridized cDNA library is passed through a heated column filled with hydroxyapatite granules. The hydroxyapatite preferentially binds to larger DNA molecules. The size of DNA that is bound is controlled by the concentration of phosphate buffer in which the cDNA library is dissolved. Thus the concentration of phosphate buffer must be tuned specifically for cDNA molecules within a certain range of sequence length. The cDNA is eluted through the column using increasing concentrations of phosphate buffer to extract increasing sizes of DNA molecules. Since the single stranded cDNA will be roughly one half the size of the re-hybridized cDNA, elution of the single stranded fraction can be managed if the mean cDNA sequence length is known. The resulting elution is intended to be enriched for the single stranded cDNA which are then amplified using PCR.

Complementary DNA (cDNA) normalization may be performed by a selective amplification method. Complementary DNA (cDNA) normalization may be performed by "Level-Up" (as described in WO2022/229128A1, which is hereby incorporated by reference). Level-Up normalization makes it possible to take a cDNA library and equalize the relative abundances of each unique transcript sequence. This is done without depletion and without targeting. In essence this allows creation of an optimal cDNA library for detecting all RNA that are present in the sample.

Thus, processing an RNA sample may comprise synthesizing cDNA using the RNA as a template and normalizing the cDNA sample by using a method of selective amplification of single stranded cDNA, the method comprising:
(i) providing a cDNA sample comprising double stranded cDNA templates, each template having a known 5' pre-attached adapter and a known 3' pre-attached adapter;
(ii) denaturing the cDNA sample to produce single stranded cDNA templates;
(iii) re-associating the cDNA sample to produce a mixture of post-association single stranded cDNA templates and post-association double stranded cDNA templates;
(iv) annealing a 5' adapter complex to the 5' pre-attached adapter of at least one post-association single stranded cDNA template, and annealing a 3' adapter complex to the 3' pre-attached adapter of the same post-association single stranded cDNA template, wherein each adapter complex comprises at least one oligonucleotide;
(v) ligating an oligonucleotide from the 5' adapter complex to the 5' pre-attached adapter of the post-association single stranded cDNA template and ligating an oligonucleotide from the 3' adapter complex to the 3' pre-attached adapter of the same post-association single stranded cDNA template; and
(vi) selectively amplifying the cDNA sample using primers specific to the ligated oligonucleotides.

In the methods, processing the RNA sample(s) may comprise synthesizing cDNA using the RNA as a template to produce double stranded cDNA templates, each cDNA template having a known 5' pre-attached adapter and a known 3' pre-attached adapter and:
(i) denaturing the cDNA to produce single stranded cDNA templates;
(ii) re-associating the cDNA to produce a mixture of post-association single stranded cDNA templates and post-association double stranded cDNA templates;
(iii) annealing a 5' adapter complex to the 5' pre-attached adapter of at least one post-association single stranded cDNA template, and annealing a 3' adapter complex to the 3' pre-attached adapter of the same post-association single stranded cDNA template, wherein each adapter complex comprises at least one oligonucleotide;
(iv) ligating an oligonucleotide from the 5' adapter complex to the 5' pre-attached adapter of the post-association single stranded cDNA template and ligating an oligonucleotide from the 3' adapter complex to the 3' pre-attached adapter of the same post-association single stranded cDNA template; and
(v) selectively amplifying the cDNA using primers specific to the ligated oligonucleotides.

In the methods:
(A) the 5' adapter complex may be a front oligonucleotide dimer comprising:
   (i) a front lig-oligonucleotide for ligating to the 5' pre-attached adapter of the (post-association) single stranded cDNA template; and
   (ii) a front link-oligonucleotide for annealing to the 5' pre-attached adapter and the front lig-oligonucleotide, the front link-oligonucleotide comprising a region complementary to the 5' pre-attached adapter and a region complementary to the front lig-oligonucleotide,
   such that, on annealing, an end of the front lig-oligonucleotide is adjacent an end of the 5' pre-attached adapter to enable ligation of the front lig-oligonucleotide to the 5' pre-attached adapter at a ligation site; and
(B) the 3' adapter complex may be a back oligonucleotide dimer comprising:
   (i) a back lig-oligonucleotide for ligating to the 3' pre-attached adapter of the (post-association) single stranded cDNA template; and
   (ii) a back link-oligonucleotide for annealing to the 3' pre-attached adapter and the back lig-oligonucleotide, the back link-oligonucleotide comprising a region complementary to the 3' pre-attached adapter and a region complementary to the back lig-oligonucleotide,
   such that, on annealing, an end of the back lig-oligonucleotide is adjacent an end of the 3' pre-attached adapter to enable ligation of the back lig-oligonucleotide to the 3' pre-attached adapter at a ligation site.

In the methods:
(A) the front link-oligonucleotide may comprise:
   (i) a template overhang region at an end of the front link-oligonucleotide proximal the region complementary to the 5' pre-attached adapter, the template overhang region being non-complementary to a corresponding region of the (post-association) single stranded cDNA template; and/or
   (ii) a lig-oligonucleotide overhang region at an end of the front link-oligonucleotide proximal the region complementary to the front lig-oligonucleotide, the lig-oligonucleotide overhang region being non-complementary to a corresponding region of the front lig-oligonucleotide; and/or
(B) the back link-oligonucleotide may comprise:
   (i) a template overhang region at an end of the back link-oligonucleotide proximal the region complementary to the 3' pre-attached adapter, the template overhang region being non-complementary to a corresponding region of the (post-association) single stranded cDNA template; and/or
   (ii) a lig-oligonucleotide overhang region at an end of the back link-oligonucleotide proximal the region complementary to the back lig-oligonucleotide, the lig-oligonucleotide overhang region being non-complementary to a corresponding region of the back lig-oligonucleotide.

In the methods, the first and second cDNA samples may comprise double stranded cDNA templates, each template having a known 5' pre-attached adapter and a known 3' pre-attached adapter;
and normalizing the first and second cDNA samples may comprise:
(i) denaturing the cDNA sample to produce single stranded cDNA templates;
(ii) re-associating the cDNA sample to produce a mixture of post-association single stranded cDNA templates and post-association double stranded cDNA templates;
(iii) annealing a 5' adapter complex to the 5' pre-attached adapter of at least one post-association single stranded cDNA template, and annealing a 3' adapter complex to the 3' pre-attached adapter of the same post-association single stranded cDNA template, wherein each adapter complex comprises at least one oligonucleotide;
(iv) ligating an oligonucleotide from the 5' adapter complex to the 5' pre-attached adapter of the post-association single stranded cDNA template and ligating an oligonucleotide from the 3' adapter complex to the 3' pre-attached adapter of the same post-association single stranded cDNA template; and
(v) selectively amplifying the cDNA sample using primers specific to the ligated oligonucleotides.

The cDNA (sample) may comprise double stranded cDNA templates, each template having a known 5' pre-attached adapter and a known 3' pre-attached adapter;
and processing (normalizing) the cDNA (sample) may comprise:
(i) denaturing the cDNA (sample) to produce single stranded cDNA templates;
(ii) re-associating the cDNA (sample) to produce a mixture of post-association single stranded cDNA templates and post-association double stranded cDNA templates;
(iii) annealing a 5' adapter complex to the 5' pre-attached adapter of at least one post-association single stranded cDNA template, and annealing a 3' adapter complex to the 3' pre-attached adapter of the same post-association single stranded cDNA template, wherein each adapter complex comprises at least one oligonucleotide;
(iv) ligating an oligonucleotide from the 5' adapter complex to the 5' pre-attached adapter of the post-association single stranded cDNA template and ligating an oligonucleotide from the 3' adapter complex to the 3' pre-attached adapter of the same post-association single stranded cDNA template; and
(v) selectively amplifying the cDNA (sample) using primers specific to the ligated oligonucleotides.

The invention also provides a method for discovering a (autoimmune) disease biomarker comprising:
(i) providing a first RNA sample extracted from a blood sample from a subject with the disease and a second RNA sample extracted from a blood sample from a subject without the disease,
(ii) synthesizing cDNA using the RNA as a template to produce first and second cDNA samples comprising double stranded cDNA templates, each template having a known 5' pre-attached adapter and a known 3' pre-attached adapter;
(iii) denaturing the first and second cDNA samples to produce single stranded cDNA templates;
(iv) re-associating the first and second cDNA samples to produce a mixture of post-association single stranded cDNA templates and post-association double stranded cDNA templates;
(v) within each of the first and second cDNA samples annealing a 5' adapter complex to the 5' pre-attached adapter of at least one post-association single stranded cDNA template, and annealing a 3' adapter complex to the 3' pre-attached adapter of the same post-association single stranded cDNA template, wherein each adapter complex comprises at least one oligonucleotide;
(vi) ligating an oligonucleotide from the 5' adapter complex to the 5' pre-attached adapter of the post-association single stranded cDNA template and ligating an oligonucleotide from the 3' adapter complex to the 3' pre-attached adapter of the same post-association single stranded cDNA template;
(vii) selectively amplifying the first and second cDNA samples using primers specific to the ligated oligonucleotides
(viii) sequencing the selectively amplified first and second cDNA samples; and
(ix) comparing the sequencing output for the first and second cDNA samples to discover a (autoimmune) disease biomarker.

The first and second RNA/cDNA samples may be kept separate and/or be separately identifiable.

The invention further provides a method for diagnosing a (autoimmune) disease in a subject, the method comprising:
(i) providing an RNA sample extracted from a blood sample obtained from the subject;
(ii) synthesizing cDNA using the RNA as a template to produce a cDNA sample comprising double stranded cDNA templates, each template having a known 5' pre-attached adapter and a known 3' pre-attached adapter;
(iii) denaturing the cDNA sample to produce single stranded cDNA templates;
(iv) re-associating the cDNA sample to produce a mixture of post-association single stranded cDNA templates and post-association double stranded cDNA templates;
(v) annealing a 5' adapter complex to the 5' pre-attached adapter of at least one post-association single stranded cDNA template, and annealing a 3' adapter complex to the 3' pre-attached adapter of the same post-association single stranded cDNA template, wherein each adapter complex comprises at least one oligonucleotide;
(vi) ligating an oligonucleotide from the 5' adapter complex to the 5' pre-attached adapter of the post-association single stranded cDNA template and ligating an oligonucleotide from the 3' adapter complex to the 3' pre-attached adapter of the same post-association single stranded cDNA template;
(vii) selectively amplifying the cDNA sample using primers specific to the ligated oligonucleotides; and
(viii) sequencing the selectively amplified cDNA sample, wherein the sequencing output is used to identify whether the subject has the (autoimmune) disease.

The invention provides method for producing a database of disease biomarkers, the method comprising:
(i) providing two or more RNA samples extracted from blood samples obtained from one or more subjects with a disease;
(ii) processing the RNA samples, optionally comprising synthesizing cDNA using the RNA as a template;
(iii) sequencing the processed RNA or cDNA, wherein the sequencing output is used to identify two or more disease biomarkers; and
(iv) compiling the disease biomarkers to form a database;
wherein processing the RNA samples comprises synthesizing cDNA using the RNA as a template to produce double stranded cDNA templates, each cDNA template having a known 5' pre-attached adapter and a known 3' pre-attached adapter and:
(i) denaturing the cDNA to produce single stranded cDNA templates;
(ii) re-associating the cDNA to produce a mixture of post-association single stranded cDNA templates and post-association double stranded cDNA templates;
(iii) annealing a 5' adapter complex to the 5' pre-attached adapter of at least one post-association single stranded cDNA template, and annealing a 3' adapter complex to the 3' pre-attached adapter of the same post-association single stranded cDNA template, wherein each adapter complex comprises at least one oligonucleotide;
(iv) ligating an oligonucleotide from the 5' adapter complex to the 5' pre-attached adapter of the post-association single stranded cDNA template and ligating an oligonucleotide from the 3' adapter complex to the 3' pre-attached adapter of the same post-association single stranded cDNA template; and
(v) selectively amplifying the cDNA using primers specific to the ligated oligonucleotides.

The two or more RNA samples may be kept separate and/or be separately identifiable.

The invention provides a method for producing an RNA vaccine for a subject with a disease, the method comprising:
(i) providing an RNA sample extracted from a blood sample obtained from the subject;
(ii) processing the RNA sample, optionally comprising synthesizing cDNA using the RNA as a template;
(iii) sequencing the processed RNA or cDNA, wherein the sequencing output is used to identify the presence of an RNA transcript sequence associated with the disease;
(iv) using the identified RNA transcript sequence to produce a first RNA vaccine for the subject;
(v) providing a further RNA sample extracted from a blood sample obtained from the subject at a later time point;
(vi) processing the further RNA sample, optionally comprising synthesizing cDNA using the RNA as a template;
(vii) sequencing the processed RNA or cDNA, wherein the sequencing output is used to identify the presence of an RNA transcript sequence associated with the disease, and
(viii) using the identified RNA transcript sequence to produce a further RNA vaccine for the subject;
wherein processing the RNA sample and further RNA sample comprises synthesizing cDNA using the RNA as a template to produce double stranded cDNA templates, each cDNA template having a known 5' pre-attached adapter and a known 3' pre-attached adapter and:
(i) denaturing the cDNA to produce single stranded cDNA templates;
(ii) re-associating the cDNA to produce a mixture of post-association single stranded cDNA templates and post-association double stranded cDNA templates;
(iii) annealing a 5' adapter complex to the 5' pre-attached adapter of at least one post-association single stranded cDNA template, and annealing a 3' adapter complex to the 3' pre-attached adapter of the same post-association single stranded cDNA template, wherein each adapter complex comprises at least one oligonucleotide;
(iv) ligating an oligonucleotide from the 5' adapter complex to the 5' pre-attached adapter of the post-association single stranded cDNA template and ligating an oligonucleotide from the 3' adapter complex to the 3' pre-attached adapter of the same post-association single stranded cDNA template; and
(v) selectively amplifying the cDNA using primers specific to the ligated oligonucleotides.

The RNA sample and further RNA sample may be kept separate and/or be separately identifiable.

In the methods:
(A) the 5' adapter complex may be a front oligonucleotide dimer comprising:
   (i) a front lig-oligonucleotide for ligating to the 5' pre-attached adapter of the (post-association) single stranded cDNA template; and
   (ii) a front link-oligonucleotide for annealing to the 5' pre-attached adapter and the front lig-oligonucleotide, the front link-oligonucleotide comprising a region complementary to the 5' pre-attached adapter and a region complementary to the front lig-oligonucleotide,
   such that, on annealing, an end of the front lig-oligonucleotide is adjacent an end of the 5' pre-attached adapter to enable ligation of the front lig-oligonucleotide to the 5' pre-attached adapter at a ligation site; and
(B) the 3' adapter complex may be a back oligonucleotide dimer comprising:
   (i) a back lig-oligonucleotide for ligating to the 3' pre-attached adapter of the (post-association) single stranded cDNA template; and
   (ii) a back link-oligonucleotide for annealing to the 3' pre-attached adapter and the back lig-oligonucleotide, the back link-oligonucleotide comprising a region complementary to the 3' pre-attached adapter and a region complementary to the back lig-oligonucleotide,
such that, on annealing, an end of the back lig-oligonucleotide is adjacent an end of the 3' pre-attached adapter to enable ligation of the back lig-oligonucleotide to the 3' pre-attached adapter at a ligation site.

In the methods:
(A) the front link-oligonucleotide may comprise:
   (i) a template overhang region at an end of the front link-oligonucleotide proximal the region complementary to the 5' pre-attached adapter, the template overhang region being non-complementary to a corresponding region of the (post-association) single stranded cDNA template; and/or
   (ii) a lig-oligonucleotide overhang region at an end of the front link-oligonucleotide proximal the region complementary to the front lig-oligonucleotide, the lig-oligonucleotide overhang region being non-complementary to a corresponding region of the front lig-oligonucleotide; and/or
(B) the back link-oligonucleotide may comprise:
   (i) a template overhang region at an end of the back link-oligonucleotide proximal the region complementary to the 3' pre-attached adapter, the template overhang region being non-complementary to a corresponding region of the (post-association) single stranded cDNA template; and/or
   (ii) a lig-oligonucleotide overhang region at an end of the back link-oligonucleotide proximal the region complementary to the back lig-oligonucleotide, the lig-oligonucleotide overhang region being non-complementary to a corresponding region of the back lig-oligonucleotide.

The template overhang and/or lig-oligonucleotide overhang may be between about 1 bp and about 20 bp in length. The template overhang and/or lig-oligonucleotide overhang may be between 2 bp and 19 bp, between 3 bp and 18 bp, between 2 bp and 17 bp, between 3 bp and 16 bp, between 2 bp and 15 bp, between 3 bp and 14 bp, between, 2 bp and 13 bp, between 3 bp and 12 bp, between 2 bp and 11 bp, between 3 bp and 10 bp, between 2 bp and 9 bp, between 3 bp and 8 bp, between 2 bp and 7 bp, between 3 bp and 6 bp, between 2 bp and 5 bp, between 3 bp and 5 bp or between 2 bp and 4 bp. Preferably, the template overhang and/or lig-oligonucleotide overhang is 3 bp.

The template overhang and/or lig-oligonucleotide overhang may be at least 2 bp, or at least 3 bp. Preferably, the template overhang and/or lig-oligonucleotide overhang is at least 3 bp.

A combined length of the front link-oligonucleotide and the front lig-oligonucleotide may be less than about 300 bp and/or a combined length of the back link-oligonucleotide and the back lig-oligonucleotide is less than about 300 bp. A combined length of the front link-oligonucleotide and the front lig-oligonucleotide may be at least about 200 bp and/or a combined length of the back link-oligonucleotide and the back lig-oligonucleotide may be at least about 100 bp.

The front and/or back link-oligonucleotide may have a length of less than 200 bp. The front and/or back link-oligonucleotide may have a length of at least 50 bp.

The front oligonucleotide dimer and/or the back oligonucleotide dimer may have at least one non-blunt end.

The front link-oligonucleotide and/or the back link-oligonucleotide may provide at least 5 bp of complementary binding either side of the ligation site.

A nucleotide sequence of the front oligonucleotide dimer may be different and non-complementary to a nucleotide sequence of the back oligonucleotide dimer.

At least one of the front oligonucleotide dimer and the back oligonucleotide dimer may be annealable to the (post-association) single stranded cDNA template at a temperature of over 30°C.

A concentration of the front oligonucleotide dimer and/or a concentration of the back oligonucleotide dimer may exceed a concentration of a predicted total single stranded cDNA concentration or total cDNA in the cDNA sample.

The step of re-associating the cDNA sample may have a duration of 0-24 hours, optionally 0-8 hours, 1-7 hours, 1-24 hours or 7-24 hours.

By using long read sequencing, it may be possible to detect full length RNA/cDNA which will provide better information for identifying the tissue source of each RNA and the specific function. While long read RNA sequencing is expensive compared to other assays, Level-Up technology makes it possible to reduce the amount of sequencing required thus lowering the overall cost.

In the methods, sequencing may comprise long read sequencing. Long read sequencing may be single-molecule long read sequencing (e.g. PacBio^{®} HiFi or Oxford Nanopore Technologies nanopore sequencing). Long read sequencing may be single-molecule nanopore sequencing. Long read sequencing may comprise tagmentation (e.g. Ilumina Complete Long Read sequencing technology). Long read sequencing may produce reads of more than 1 kb, more than 5kb, more than 10kb or more than 20kb.

The RNA may be full-length RNA. Thus, the processed RNA or cDNA that is sequenced may be full length. By "full length" is meant that the sequence of the whole length (or at least 99%, 98%, 95%, 90% or 80% of the length) of the RNA/cDNA molecule may be obtained i.e. RNA or cDNA molecules are not fragmented before sequencing. Entire spliced isoforms may be directly observed. The methods may not comprise a step of (actively) fragmenting RNA and/or cDNA prior to sequencing.

Sequencing may comprise the use of long-read, full-length RNA sequencing. This allows for direct observation of entire spliced isoforms.

Level-Up makes it possible to analyze samples with RNA degradation by increasing the presence of low abundance transcripts. However, it is preferable if RNA degradation is minimized during the processing of biological samples. The present inventors have developed a method for processing a blood sample when RNA extraction is not carried out on the day of blood collection. Specific steps for blood sample freezing, storage and thawing improve the condition of samples, particularly if they are to be subjected to long read sequencing.

The invention provides a method for processing a blood sample comprising:
(i) storing the blood sample at -15°C or below;
(ii) thawing the blood sample at 5 to 30°C for at least 1 hour; and
(iii) extracting RNA from the thawed blood sample.

The blood sample may be a liquid (i.e. non-dried) blood sample. The blood sample may be whole blood. The blood sample may be in a sample tube. The blood sample may not absorbed into a material such as a sponge.

The blood sample may be stored at between -15°C and -80°C, -15°C and -70°C, -15°C and - 60°C, -15°C and -50°C, -15°C and -40°C, -15°C and -30°C or -15°C and -20°C.

The blood sample may be stored at -15°C or below within 12 hours, 8 hours, 5 hours, 2 hours, 1 hour, 30 minutes, 15 minutes, 5 minutes or 1 minute of collection. Preferably, the blood sample is stored at -15°C or below within 12 hours of collection (i.e. taking the blood sample from the subject).

The blood sample may be stored at -20°C or below. The blood sample may be stored at - 20°C or below within 12 hours, 8 hours, 5 hours, 2 hours, 1 hour, 30 minutes, 15 minutes, 5 minutes or 1 minute of collection. Preferably, the blood sample is stored at -20°C or below within 12 hours of collection (i.e. taking the blood sample from the subject).

The blood sample may be stored at -15°C or below (optionally -20°C or below) for at least 24 hours (and optionally for no more than 72 hours, 1 week, 2 weeks, 4 weeks, 1 month or 2 months) before storing at -70°C or below (optionally -80°C or below) for no more than 4, 5, 6, 7, 8, 9, 10, 11, or 12 months or 2, 3, 4 or 5 years. Preferably, storage at -70°C or below (optionally -80°C or below) is for no more than 5 years.

Preferably, thawing of the blood sample takes place on the same day RNA is to be extracted. The blood sample may be thawed at 16 to 29°C, 17 to 28°C, 18 to 27°C, 18 to 26°C or 18 to 25°C. Preferably, the blood sample is thawed at 18 to 25°C. The duration of the thawing step may be 1 to 5 hours, 2 to 5 hours, 1 to 4 hours, 2 to 4 hours, 1 to 3 hours, 2 to 3 hours or 1 to 2 hours. The blood sample may be thawed at 18 to 25°C for 1 to 3 hours. More preferably, the blood sample is thawed at 18 to 25°C for 3 hours.

Once the blood sample is fully thawed the sample tube may be inverted at least 5, 6, 7, 8, 9 or 10 times. Preferably, the sample tube is inverted 10 times. The blood sample may then be incubated at 18 to 25°C for around 2 hours prior to RNA extraction.

RNA may be extracted from the (thawed) blood sample using the Qiagen Paxgene Blood RNA Kit.

In the methods, prior to step (i), the blood may be received in a container (optionally a Paxgene Blood RNA Tube) at room temperature (5 to 30°C, preferably 18 to 25°C). The container may be inverted at least 5, 6, 7, 8, 9 or 10 times immediately after blood collection. Preferably, the container is inverted 10 times immediately after blood collection. The blood sample may be stored at -15°C or below (or -20°C or below) immediately after being inverted.

Thus, the invention provides a method for processing a blood sample comprising:
(i) receiving the blood sample in a container at 18 to 25°C;
(ii) storing the blood sample at -20°C or below within 12 hours of collection;
(iii) thawing the blood sample at 18 to 25°C for 1 to 3 hours; and
(iv) extracting RNA from the thawed blood sample.

The blood sample may be no more than 5 ml, 3 ml, 2.5 ml or 2 ml. Preferably, the blood sample is no more than 2.5 ml. The blood sample may be between 0.1 ml and 5 ml, 0.5 ml and 5 ml, 1 ml and 4 ml, or 2 ml and 3 ml.

The methods for processing a blood sample may be combined with the methods outlined above that employ an RNA or cDNA sample. Thus, the RNA or cDNA sample may be obtained from blood by following the steps of the methods for processing a blood sample described herein. In addition, the method may comprise a step of synthesizing cDNA using the extracted RNA as a template (i.e. converting the extracted RNA into cDNA, reverse transcription). The first RNA/cDNA sample and the second RNA/cDNA sample may be obtained from blood by following the steps of the methods for processing a blood sample described herein. In addition, the method may comprise a step of synthesizing cDNA using the extracted RNA as a template (i.e. converting the extracted RNA into cDNA, reverse transcription).

The invention provides a method for discovering a (autoimmune) disease biomarker comprising:
(i) receiving a first blood sample from a subject with a disease in a first container and a second blood sample from a subject without the disease in a second container at 18 to 25°C;
(ii) storing the first and second blood samples at -20°C or below within 12 hours of collection from the subjects;
(iii) thawing the first and second blood samples at 18 to 25°C for 1 to 3 hours;
(iv) extracting RNA from the thawed first and second blood samples;
(v) synthesizing cDNA using the extracted RNA as a template to form a first cDNA sample from the subject with the disease and a second cDNA sample from the subject without the disease;
(vi) processing (normalizing) the first and the second cDNA samples;
(vii) sequencing the processed (normalized) first and second cDNA samples; and
(viii) comparing the sequencing output for the first and second cDNA samples to discover a disease biomarker.

The invention provides a method for diagnosing a (autoimmune) disease in a subject comprising:
(i) receiving a blood sample from the subject in a container at 18 to 25°C;
(ii) storing the blood sample at -20°C or below within 12 hours of collection from the subject;
(iii) thawing the blood sample at 18 to 25°C for 1 to 3 hours;
(iv) extracting RNA from the thawed blood sample;
(v) synthesizing cDNA using the extracted RNA as a template to form a cDNA sample from the subject;
(vi) processing (normalizing) the cDNA sample; and
(vii) sequencing the processed (normalized) cDNA sample, wherein the sequencing output is used to identify whether the subject has the disease.

The invention provides use in a method for discovering an autoimmune disease biomarker and/or a set of RNA sequences associated with a disease (optionally, wherein each of the set of RNA sequences encodes an antigenic peptide) of an oligonucleotide dimer composition for selective amplification of single stranded cDNA by ligation of an oligonucleotide to a 5' and a 3' end of a post-association single stranded cDNA template having known 5' and 3' pre-attached adapters, wherein the composition comprises:
(A) a front oligonucleotide dimer comprising:
   (i) a front lig-oligonucleotide for ligating to the 5' pre-attached adapter of the post-association single stranded cDNA template; and
   (ii) a front link-oligonucleotide for annealing to the 5' pre-attached adapter and the front lig-oligonucleotide, the front link-oligonucleotide comprising a region complementary to the 5' pre-attached adapter and a region complementary to the front lig-oligonucleotide,
   such that, on annealing, an end of the front lig-oligonucleotide is adjacent an end of the 5' pre-attached adapter to enable ligation of the front lig-oligonucleotide to the 5' pre-attached adapter at a ligation site; and
(B) a back oligonucleotide dimer comprising:
   (i) a back lig-oligonucleotide for ligating to the 3' pre-attached adapter of the post-association single stranded cDNA template; and
   (ii) a back link-oligonucleotide for annealing to the 3' pre-attached adapter and the back lig-oligonucleotide, the back link oligonucleotide comprising a region complementary to the 3' pre-attached adapter and a region complementary to the back lig-oligonucleotide,
   such that, on annealing, an end of the back lig-oligonucleotide is adjacent an end of the 3' pre-attached adapter to enable ligation of the back lig-oligonucleotide to the 3' pre-attached adapter at a ligation site.

The invention also provides use in a method for diagnosing and/or prognosing an autoimmune disease in a subject of an oligonucleotide dimer composition for selective amplification of single stranded cDNA by ligation of an oligonucleotide to a 5' and a 3' end of a post-association single stranded cDNA template having known 5' and 3' pre-attached adapters, wherein the composition comprises:
(A) a front oligonucleotide dimer comprising:
   (i) a front lig-oligonucleotide for ligating to the 5' pre-attached adapter of the post-association single stranded cDNA template; and
   (ii) a front link-oligonucleotide for annealing to the 5' pre-attached adapter and the front lig-oligonucleotide, the front link-oligonucleotide comprising a region complementary to the 5' pre-attached adapter and a region complementary to the front lig-oligonucleotide,
   such that, on annealing, an end of the front lig-oligonucleotide is adjacent an end of the 5' pre-attached adapter to enable ligation of the front lig-oligonucleotide to the 5' pre-attached adapter at a ligation site; and
(B) a back oligonucleotide dimer comprising:
   (i) a back lig-oligonucleotide for ligating to the 3' pre-attached adapter of the post-association single stranded cDNA template; and
   (ii) a back link-oligonucleotide for annealing to the 3' pre-attached adapter and the back lig-oligonucleotide, the back link oligonucleotide comprising a region complementary to the 3' pre-attached adapter and a region complementary to the back lig-oligonucleotide,
   such that, on annealing, an end of the back lig-oligonucleotide is adjacent an end of the 3' pre-attached adapter to enable ligation of the back lig-oligonucleotide to the 3' pre-attached adapter at a ligation site.

The oligonucleotide dimer composition as defined herein may also be used in a method for characterising a disease in a subject, a method for selecting a treatment for a disease in a subject and/or a method for predicting the responsiveness of a subject with a disease to a therapeutic agent. The disease may be an autoimmune disease.

Herein:
(A) the front link-oligonucleotide may comprise:
   (i) a template overhang region at an end of the front link-oligonucleotide proximal the region complementary to the 5' pre-attached adapter, the template overhang region being non-complementary to a corresponding region of the post-association single stranded cDNA template; and/or
   (ii) a lig-oligonucleotide overhang region at an end of the front link-oligonucleotide proximal the region complementary to the front lig-oligonucleotide, the lig-oligonucleotide overhang region being non-complementary to a corresponding region of the front lig-oligonucleotide; and/or
(B) the back link-oligonucleotide may comprise:
   (i) a template overhang region at an end of the back link-oligonucleotide proximal the region complementary to the 3' pre-attached adapter, the template overhang region being non-complementary to a corresponding region of the post-association single stranded cDNA template; and/or
   (ii) a lig-oligonucleotide overhang region at an end of the back link-oligonucleotide proximal the region complementary to the back lig-oligonucleotide, the lig-oligonucleotide overhang region being non-complementary to a corresponding region of the back lig-oligonucleotide.

The template overhang and/or lig-oligonucleotide overhang may be between about 1 bp and about 20 bp in length. The template overhang and/or lig-oligonucleotide overhang may be between 2 bp and 19 bp, between 3 bp and 18 bp, between 2 bp and 17 bp, between 3 bp and 16 bp, between 2 bp and 15 bp, between 3 bp and 14 bp, between, 2 bp and 13 bp, between 3 bp and 12 bp, between 2 bp and 11 bp, between 3 bp and 10 bp, between 2 bp and 9 bp, between 3 bp and 8 bp, between 2 bp and 7 bp, between 3 bp and 6 bp, between 2 bp and 5 bp, between 3 bp and 5 bp or between 2 bp and 4 bp. Preferably, the template overhang and/or lig-oligonucleotide overhang is 3 bp.

The template overhang and/or lig-oligonucleotide overhang may be at least 2 bp, or at least 3 bp. Preferably, the template overhang and/or lig-oligonucleotide overhang is at least 3 bp.

A combined length of the front link-oligonucleotide and the front lig-oligonucleotide may be less than about 300 bp and/or a combined length of the back link-oligonucleotide and the back lig-oligonucleotide is less than about 300 bp.

The front and/or back link-oligonucleotide may have a length of less than 200 bp.

The front oligonucleotide dimer and/or the back oligonucleotide dimer may have at least one non-blunt end.

In use of the composition, the front link-oligonucleotide and/or the back link-oligonucleotide may provide at least 5 bp of complementary binding either side of the ligation site.

A nucleotide sequence of the front oligonucleotide dimer may be different and non-complementary to a nucleotide sequence of the back oligonucleotide dimer.

The front oligonucleotide dimer and/or the back oligonucleotide dimer may be annealable to the post-association single stranded cDNA template at a temperature of over 30°C.

A selective amplification kit is provided for selectively amplifying low abundance RNA/cDNA from a RNA/cDNA sample and/or for selective amplification of cDNA comprising known adapter sequences, the cDNA sample comprising cDNA templates having known 5' and 3' pre-attached adapters, the kit comprising means for preparing an oligonucleotide dimer composition as described above and means for implementing the method of selective amplification as described above.

The invention provides use of kit as described herein in a method for diagnosing and/or prognosing a disease in a subject. Also provided is use of kit as described herein in a method for characterising a disease in a subject, a method for selecting a treatment for a disease in a subject and/or a method for predicting the responsiveness of a subject with a disease to a therapeutic agent. The disease may be an autoimmune disease.

A selective amplification kit is also provided for selectively amplifying low abundance RNA/cDNA from a first RNA/cDNA sample from a subject with a disease and a second RNA/cDNA sample from a subject without the disease and/or for selective amplification of cDNA comprising known adapter sequences, the first and second cDNA samples comprising cDNA templates having known 5' and 3' pre-attached adapters, the kit comprising means for preparing an oligonucleotide dimer composition as described above and means for implementing the method of selective amplification as described above.

The invention provides use of kit as described herein in a method for discovering a disease biomarker and/or a set of RNA sequences associated with a disease (optionally, wherein each of the set of RNA sequences encodes an antigenic peptide).

The means for preparing an oligonucleotide dimer composition may comprise a front lig-oligonucleotide, a front link-oligonucleotide, a back lig-oligonucleotide and/or a back link-oligonucleotide as described herein. The means for preparing an oligonucleotide dimer composition may comprise a front oligonucleotide dimer and/or a back oligonucleotide dimer as described herein.

The means for implementing the method of selective amplification may comprise primers specific to the front and/or back lig-oligonucleotides.

The kit may further comprise a hybridization buffer. The hybridization buffer may comprise HEPES 1M (pH = 7.5), NaCl 5M and H₂0. The kit may also comprise ligase and/or ligase buffer. Any suitable ligase may be used. The ligase may be a nick repair ligase or a blunt end ligase. Optionally, the ligase may be Taq DNA ligase. Suitable ligase buffers are also well known and commercially available.

The kit may further comprise primers for adding phosphate groups to cDNA prior to its use as a cDNA sample. These primers are based on the known 5' pre-attached adapter and known 3' pre-attached adapter sequences.

The kit may further comprise suitable reagents for PCR including one or more, up to all, of a polymerase, dinucleotide triphosphates (dNTPs), MgCl₂ and buffer. Any suitable polymerase may be utilised. Generally, DNA polymerases are used to amplify nucleic acid targets according to the invention. Examples include thermostable polymerases such as Taq or Pfu polymerase and the various derivatives of those enzymes. Suitable buffers are also well known and commercially available and may be included in a PCR mastermix that includes the majority of the components required for PCR amplification.

The kit may further comprise suitable reagents for reverse transcription of RNA to cDNA including a reverse transcriptase enzyme. Any suitable reverse transcriptase may be utilised. Suitable buffers are also well known and commercially available and may be included in a reverse transcription mastermix that includes the majority of the components required for reverse transcription.

The kit may further comprise suitable reagents for processing a blood sample, for example a container (optionally a PAXgene Blood RNA tube), RNA stabilizing reagent and/or blood cell lysis buffer. The reagents may be RNase-free. RNA stabilizing reagents are commercially available and include RNAlater^{®} (Sigma-Aldrich) and RNAprotect (Qiagen). A suitable RNA stabilizing reagent may comprise EDTA, sodium citrate and/or ammonium sulfate, for example 70% (w/v) Ammonium Sulfate, 25 mM Sodium Citrate and/or 10 mM EDTA. The pH may be adjusted to 5.2 with sulfuric acid.

The invention provides use of a set of reagents in a method as described herein, the set of reagents comprising:
a front lig-oligonucleotide, a front link-oligonucleotide, a back lig-oligonucleotide and/or a back link-oligonucleotide; and
primers specific to the front and/or back lig-oligonucleotides.

The front lig-oligonucleotide, front link-oligonucleotide, back lig-oligonucleotide and/or back link-oligonucleotide may be provided as an oligonucleotide dimer composition as described herein.

The set of reagents may further comprise one or more up to all of the following: a hybridization buffer (optionally comprising HEPES 1M (pH = 7.5), NaCl 5M and H₂0), a ligase, a ligase buffer, a primer pair for adding phosphate groups to cDNA, a DNA polymerase and/or dNTPs.

The set of reagents may further comprise suitable reagents for processing a blood sample, for example a container (optionally a PAXgene Blood RNA tube), RNA stabilizing reagent and/or blood cell lysis buffer. The reagents may be RNase-free. RNA stabilizing reagents are commercially available and include RNAlater^{®} (Sigma-Aldrich) and RNAprotect (Qiagen). A suitable RNA stabilizing reagent may comprise EDTA, sodium citrate and/or ammonium sulfate, for example 70% (w/v) Ammonium Sulfate, 25 mM Sodium Citrate and/or 10 mM EDTA. The pH may be adjusted to 5.2 with sulfuric acid. Incubation for cell lysis can be, for example, 1 minute to 3 hours.

The set of reagents may comprise:
a RNA stabilizing reagent;
a (blood) cell lysis buffer;
a front lig-oligonucleotide, a front link-oligonucleotide, a back lig-oligonucleotide and/or a back link-oligonucleotide;
primers specific to the front and/or back lig-oligonucleotides;
a hybridization buffer;
a ligase;
a ligase buffer; and
a primer pair for adding phosphate groups to cDNA.

The oligonucleotide dimer composition for selective amplification of single stranded cDNA described herein may have the ability to select cDNA having known adapter sequences. This may be applied for single cell sequencing where adapters are necessary for assigning reads to individual cells. In this application, cDNA sequences without cell identifying barcodes/adapters arise within the cDNA library. These are known as template switching oligo (TSO) artefacts and are undesirable in single cell sequencing projects due to not being assignable to a cell of origin. The present method can be applied to only select for cDNA sequences with the desired adapter sequences thus effectively limiting the sequencing of TSO artefacts. The present method can also be performed with single cell cDNA libraries to both remove TSO artefacts and to improve transcriptome coverage per cell.

TSO clean-up can also be carried out without normalization in which case it is not necessary to include a step of re-associating the cDNA sample to produce a mixture of post-association single stranded cDNA templates and post-association double stranded cDNA templates.

The invention provides a method for diagnosing a disease in a subject, the method comprising:
(i) providing an RNA sample extracted from a blood sample obtained from the subject;
(ii) synthesizing cDNA using the RNA as a template to produce a cDNA sample comprising double stranded cDNA templates, a portion of the templates having a known 5' pre-attached adapter and a known 3' pre-attached adapter;
(iii) denaturing the cDNA sample to produce single stranded cDNA templates;
(iv) annealing a 5' adapter complex to the 5' pre-attached adapter of at least one single stranded cDNA template, and annealing a 3' adapter complex to the 3' pre-attached adapter of the same single stranded cDNA template, wherein each adapter complex comprises at least one oligonucleotide;
(v) ligating an oligonucleotide from the 5' adapter complex to the 5' pre-attached adapter of the single stranded cDNA template and ligating an oligonucleotide from the 3' adapter complex to the 3' pre-attached adapter of the same single stranded cDNA template;
(vi) selectively amplifying the cDNA sample using primers specific to the ligated oligonucleotides; and
(vii) sequencing the cDNA sample, wherein the sequencing output is used to identify whether the subject has the disease.

The invention provides a method for discovering a disease biomarker, the method comprising:
(i) providing a first RNA sample extracted from a blood sample obtained from a subject with the disease and a second RNA sample extracted from a blood sample obtained from a subject without the disease;
(ii) synthesizing cDNA using the RNA as a template to produce a first cDNA sample and a second cDNA sample, the samples comprising double stranded cDNA templates, a portion of the templates having a known 5' pre-attached adapter and a known 3' pre-attached adapter
(iii) denaturing each cDNA sample to produce single stranded cDNA templates;
(iv) annealing a 5' adapter complex to the 5' pre-attached adapter of at least one single stranded cDNA template, and annealing a 3' adapter complex to the 3' pre-attached adapter of the same single stranded cDNA template, wherein each adapter complex comprises at least one oligonucleotide;
(v) ligating an oligonucleotide from the 5' adapter complex to the 5' pre-attached adapter of the single stranded cDNA template and ligating an oligonucleotide from the 3' adapter complex to the 3' pre-attached adapter of the same single stranded cDNA template;
(vi) selectively amplifying each cDNA sample using primers specific to the ligated oligonucleotides; and
(vii) sequencing each cDNA sample and comparing the sequencing output for the first and second cDNA samples to discover a disease biomarker.

The disease may be an autoimmune disease. The RNA/cDNA sample may be from a single cell.

The oligonucleotide dimer composition is suitable for use in a method as defined herein for selective amplification of cDNA comprising known adapter sequences. The method of selective amplification of cDNA comprising known adapter sequences as defined herein can be used for discovery of a disease biomarker and/or a set of RNA sequences associated with a disease or in a process of diagnosing and/or prognosing a disease. The kits and sets of reagents defined herein are also suitable for use in the method for selective amplification of cDNA comprising known adapter sequences.

The cDNA sample may comprise no more than 800ng, 700ng, 500ng, 100ng, 20ng, 10ng, 5ng or 1ng of starting cDNA. The cDNA sample may comprise 1-800 ng, 1-500ng, 5-100ng, or 10-50ng of starting cDNA.

RNA from a sample may firstly be reverse transcribed to cDNA. Sample types include blood samples (in particular from plasma, and also serum), other bodily fluids such as saliva, urine or lymph fluid. Other sample types include solid tissues, including frozen tissue or formalin fixed, paraffin embedded (FFPE) material. The RNA may be messenger RNA (mRNA), microRNA (miRNA) etc. The RNA may be reverse transcribed using a reverse transcriptase enzyme to form a complementary DNA (cDNA) molecule. Methods for reverse transcribing RNA to cDNA using a reverse transcriptase are well-known in the art. Any suitable reverse transcriptase can be used, examples of suitable reverse transcriptases being widely available in the art. The initial cDNA molecule may be single stranded until DNA polymerase has been used to generate the complementary strand. Commercially available kits (such as NEBNext ^{®} Single Cell/Low Input cDNA Synthesis & Amplification Module) can be used to convert RNA into double stranded cDNA with 5' and 3' adapters. Primers based on the 5' and 3' adapters can be used to add phosphate groups to the cDNA. A cDNA purification step (for example with ProNex or Ampure beads) may be carried out prior to use of the cDNA as a cDNA sample or prior to sequencing.

As the methods only require a small amount of starting cDNA, this can be produced from a small quantity of RNA and/or without the requirement for additional PCR cycles during the generation of the cDNA. The RNA sample may comprise no more than 3.5µg, 3µg, 2µg, 1µg, 500ng, 100 ng, 10ng or 1 ng of starting RNA. The RNA sample may comprise 1ng-3µg, 10ng-2µg, or 100ng-1µg of starting RNA.

The invention provides a system or test kit for discovering a disease biomarker and/or set of RNA sequences associated with a disease, comprising:
(a) one or more testing devices for processing (normalizing) a first RNA/cDNA sample from a subject with a disease (cancer) and a second RNA/cDNA sample from a subject without the disease (cancer) and sequencing the processed (normalized) first and second RNA/cDNA samples;
(b) a processor; and
(c) storage medium comprising a computer application that, when executed by the processor, is configured to:
   (i) access the determined sequence(s) for the first and second RNA/cDNA samples on the one or more testing devices;
   (ii) calculate whether there is a RNA/cDNA sequence that is present in the first RNA/cDNA sample but not in the second RNA/cDNA sample or is present in the second RNA/cDNA sample but not in the first RNA/cDNA sample; and
   (iii) output from the processor the result of step (ii).

The invention provides a system or test kit for diagnosing and/or prognosing a disease in a subject, comprising:
(a) one or more testing devices for processing (normalizing) a RNA/cDNA sample from a subject and sequencing the processed (normalized) cDNA sample
(b) a processor; and
(c) storage medium comprising a computer application that, when executed by the processor, is configured to:
   (i) access the determined sequence(s) for the RNA/cDNA sample on the one or more testing devices
   (ii) calculate whether a RNA/cDNA sequence is present or absent, wherein the presence or absence of the RNA/cDNA sequence is associated with the disease; and
   (iii) output from the processor whether a subject has the disease and/or a prognosis for the disease.

The one or more testing devices may use/comprise an oligonucleotide dimer composition as described herein. The one or more testing devices may comprise a long-read sequencer.

The system or test kit may further comprise a display for the output from the processor.

There is also provided a computer application or storage medium comprising a computer application as defined herein.

The invention provides use of a method, device or kit as described herein in a method for determining soil microfauna composition.

Soil microfauna composition may be determined through the identification of microorganisms and/or viruses present in a soil sample. This may be achieved through the identification of sequences in the sequencing output.

The invention provides use of a method, device or kit as described herein in ecological research.

Ecological research may comprise sequencing processed RNA or cDNA from one or more species. Ecological research may comprise obtaining information regarding the transcriptome of one or more species. Ecological research may lead to improved understanding of the overall biology of one or more species and/or how one or more species impact their local ecology.

The invention provides use of a method, device or kit as described herein in a method for assessing water quality.

Water quality may be assessed through the identification/detection of microorganisms (for example bacteria and/or fungi) and/or viruses. Water quality may be assessed by extracting RNA from a water sample from a water source. This may be achieved through the identification of sequences in the sequencing output.

The invention provides use of a method, device or kit as described herein in a method for screening for dangerous biological material.

The dangerous biological material may comprise a fungus, bacterium and/or a virus, optionally a pathogenic fungus, bacterium or virus, and/or a fungal, plant or animal derived toxin or drug that may still contain traces of nucleic acid (for example RNA). The screening may take place at a travel gateway.

The invention provides use of a method, device or kit as described herein in a method for confirming the identity of a subject.

Confirming the identify of a subject may be achieved through the identification of sequences in the sequencing output. Confirming the identity of a subject may comprise DNA fingerprinting or include steps from a DNA fingerprinting method. Confirming the identify of a subject may be achieved through analysis of repetitive sequences that are highly variable, for example variable number tandem repeats, optionally short tandem repeats. The method may take place at a travel gateway.

### DETAILED DESCRIPTION

The invention will now be described in further detail, by way of example only, with reference to the following examples and the accompanying figures, in which:
Figure 1 is a schematic overview showing addition of ligation sequences to the ends of single stranded cDNA templates;
Figure 2 is a schematic overview of an embodiment of a cDNA normalization process in accordance with the present invention;
Figure 3 is a schematic overview of Front and Back oligonucleotide dimer structures as shown annealed to single strand cDNA template, Fig 3A is a detail thereof and Fig 3B provides an illustration using the Example sequence representations recited herein;
Figure 4 is a graph showing length distribution from gel electrophoresis of input cDNA;
Figure 5 is a graph showing length distribution from gel electrophoresis of normalized cDNA resulting from a cDNA normalisation process as shown in Figure 2; and
Figure 6 shows saturation curves for input cDNA and a normalized cDNA normalized as in Figure 2 using Nanopore cDNA sequencing.
Figure 7 is a 2D PCA-plot from 13573 transcripts showing clustering of cancer and control samples in the dataset.

The invention is based on methods that take advantage of the ability to generate full length sequences from RNA extracted from blood without fragmenting RNA or cDNA products before sequencing. This provides a transcriptome representing any RNA that make its way into the circulatory system including RNA from typical blood cells (like red blood cells, white blood cells, other immune cells, etc.), RNA from any other cells that are typically uncommon in the circulatory system such as cancer cells or cells that somehow dislodged into the circulatory system and extracellular RNA which could have originated from any cell within the body. By detecting RNA from all these sources and at full length the present inventors can ascribe each RNA to a cell/tissue of origin as well as a state of cell or tissue behaviour. The transcription start site, end site, and splicing are features which typically represent unique combinations used by different cell types. This information can also be used to directly identify the protein isoform that would be translated from messenger RNA. The ability to detect low level signals of full length RNA which can then be translated into protein isoforms means the present inventors can detect unique protein isoforms that are expressed exclusively by certain cells, for example cancer cells. These isoforms will have sections of their peptide sequence which are unique to the specific cells, for example the cancer cells. These cancer specific peptide sub-sequences are also known as neoantigens because they are typically presented on the cell surface via MHC complexes.

Detecting isoform based neoantigens, as opposed to mutation based neoantigens, has particular advantages. For example, it means that instead of having to design a new vaccine for each patient based on the unique mutation presenting in each patient, the present methods make it possible to identify a set of isoform based neoantigens that are represented across a large percentage of the population. This means that RNA cancer vaccines can be based on that set instead of having to design new vaccines for each patient. With this paradigm, the development, safety testing, production, QC, and delivery of RNA cancer vaccines can be managed much more economically and with less risk of negative effects. This means that RNA cancer vaccines can be more affordable and also means that RNA cancer vaccines could be used more often since they would be safer and more cost effective.

The use of blood samples rather than tumour samples to find the neoantigen targets is also advantageous. With tumour samples there is a limited amount of material that may be difficult to obtain and only available at a particular time point. This also means targets are based on the removed primary cells when the aim is to target the remaining distal cells that are likely to be genetically different. If targets are not identified that span all remaining cancer cells it creates selective pressure to allow cancer cells that do not present the targets to thrive and cause recurrence. This may even encourage the development of cancers with higher mutations rates that could be more problematic.

As the present methods allow for continuous monitoring of patients for neoantigen targets and because they could help make RNA cancer vaccines cheaper and safer, RNA cancer vaccine therapy could be used early and often. It can be applied multiple times depending on the neoantigen readouts obtained from the present methods until it is observed that there are no longer any neoantigens presenting in the blood of the patient.

Neoantigen targets also represent unique structures in cancer proteins that could be exploited for new drug development. Since a large percentage of targeted cancer therapies interact with proteins, data obtained from the present methods could be used to inform usefulness of a wide range of cancer therapies, including check point inhibitors.

The present methods can detect diseases at the earliest stages and indicate best therapies all from one testing paradigm. This allows for not only earlier treatment of diseases thus increasing success rates but also reducing time to treatment after diagnosis. The process could also be applied to prevent the formation of tumours or damaging cancers by screening the population and applying RNA cancer vaccines before cancer cells have the chance to grow to any meaningful size.

The data collected using the present methods can be used to compile an extensive full length RNA human database which can be used to data mine new potential drug targets.

In summary, unlike with mutation based methods which have to be designed for each patient, the present methods use isoform level targets which should be common across patients. This means it is possible to identify a set of targets that would work for a large percentage of the population. So RNA cancer vaccines can be developed, tested, and produced for each of the targets in the set of targets and applied individually or in combination when detected using the methods herein. This vastly reduces the cost and increases the safety which means RNA cancer vaccines could be used early and often. This means that that it is not necessary to destroy all the cancer cells in one go. The present methods allow for monitoring and adjusting treatment until all neoantigens in the blood disappear. The present methods could also guide complementary treatments like checkpoint inhibitors so that combination therapies could be applied when needed. Thus, the present methods could be applied to both prevent cancer formation and treat already formed cancer.

These concepts could be applied in a very similar way with a wide range of diseases including autoimmune, diabetes, coronary, metabolic, and others.

### cDNA normalization

RNA or cDNA samples are typically dominated by sequences from highly expressed genes. Normalization to achieve a more uniform distribution of sequences can increase the efficiency of sequencing for transcript discovery and/or detection. First, genes and isoforms which are specific to the condition in question are easier to detect, and second, there is less redundancy in data generated reducing data storage requirements.

Thus, the methods provided herein may include a processing step in the form of cDNA normalization. To address issues in current cDNA normalization technology, the inventor has developed an improved selective amplification method (termed "Level-Up"). This method utilises the same denaturation and re-hybridization process as the DSN and column methods described herein. However, the present method differs from other approaches by using a non-depletion or additive mechanism. In other words, Level-Up provides a method and means for increasing the amount of low abundance cDNA in a sample. These methods and means can be used for cDNA normalization in sequencing processes or for other processes that would benefit from amplification of low abundance cDNA, such as discovery, detection or identification of biomarkers and production of RNA vaccines.

In the context of the present invention, the following explanations of terms and methods are provided to better describe the present disclosure and to provide guidance in the practice of the present disclosure.

The phrase 'selective amplification' is used to describe the method developed by the inventor of amplification of particular DNA templates in preference to other DNA templates, for example, amplification of only single stranded cDNA in a sample that comprises a mixture of single stranded and double stranded cDNA. The term is also used herein to describe preferentially amplifying a particular category of DNA, such as low abundance DNA.

The term 'adapter' is used to describe a short DNA sequence added to an end of a DNA template, such as those commonly used in RNA sequencing by ligating an adapter to a cDNA template. A `3' pre-attached adapter' refers to an adapter having a known nucleotide sequence that has been added to the 3' end of a cDNA template. A '5' pre-attached adapter' refers to an adapter having a known nucleotide sequence that has been added to the 5' end of a cDNA template.

The terms 'normalized' and 'normalized fraction' refer to the process of levelling the abundance of different transcripts within a sample. This can be achieved by prior art methods of reducing the amount of highly abundant transcripts, or by using the methods described herein to selectively amplify low abundance transcripts.

The phrase 'post-association single stranded cDNA template' as used in the context of the present invention, refers to a single stranded cDNA template that has been generated by disassociating and re-associating (i.e., denaturing and re-hybridizing) a sample of double stranded cDNA to form a mixture of single stranded cDNA and double stranded cDNA. The single stranded cDNA that remains single stranded after re-association is referred to as post-association single stranded cDNA. If a re-association step is not carried out, the phrase 'post-association single stranded cDNA template' is interchangeable with 'single stranded cDNA template' in the embodiments defined herein.

The phrase `ligated-adapter-cDNA template' as used herein, refers to a cDNA template formed by ligation of an adapter to a cDNA template.

The present invention encompasses an 'adapter complex' which is suitable for annealing to an end of a post-association single stranded cDNA template. The term 'adapter complex' refers to an adapter that comprises more than one component.

The terms 'front oligonucleotide dimer', 'front k-linker' and 'front dimer' as used in the context of the present invention, refer to an adapter complex that can be annealed to the 5' end of a post-association single stranded cDNA template. The terms 'back oligonucleotide dimer', 'back k-linker' and 'back dimer', as used in the context of the present invention, refer to an adapter complex that can be annealed to the 3' end of a post-association single stranded cDNA template.

The terms 'front lig-oligonucleotide' and 'front lig' as used in the context of the present invention, refer to an oligonucleotide component of the front dimer. The terms 'back lig-oligonucleotide' or 'back lig' as used in the context of the present invention, refer to an oligonucleotide component of the back dimer.

The terms 'front link-oligonucleotide' and 'front link' as used in the context of the present invention, refer to an oligonucleotide component of the front dimer. The terms 'back link-oligonucleotide' and 'back link' as used in the context of the present invention, refer to an oligonucleotide component of the back dimer.

The term 'overhang' is used in the context of the present invention to describe an overhanging region of the sequence of the dimer of the present invention, where the overhanging region is non-complementary to the region with which it is paired, once annealed to a post-association single stranded cDNA template, such that the overhanging region does not bind with its paired region.

The single stranded cDNA is selectively amplified using primers specific to the ligated oligonucleotides. As single stranded DNA is the template, the primer region of one primer of a specific primer pair is complementary to the single stranded DNA molecule. The other primer of the specific primer pair comprises a primer region which is complementary to, and therefore hybridises with, the complementary single stranded DNA molecule formed during an amplification cycle. Thus, one primer is complementary to one of the ligated oligonucleotides and the other primer comprises (at least partially) the sequence of the other ligated oligonucleotide.

### Previously developed methods of cDNA normalization

There are two forms of full length cDNA normalization that have been previously developed: the Duplex Specific Nuclease (DSN) method (Zhulidov, P. A. et al. Simple cDNA normalization using kamchatka crab duplex-specific nuclease. Nucleic Acids Res. 32, e37 (2004)) and the hydroxyapatite column method (Andrews-Pfannkoch, C., Fadrosh, D. W., Thorpe, J. & Williamson, S. J. Hydroxyapatite-mediated separation of double-stranded DNA, single-stranded DNA, and RNA genomes from natural viral assemblages. Appl. Environ. Microbiol. 76, 5039-5045 (2010)). Both methods rely on the denaturation and re-hybridization of cDNA strands. As the single stranded cDNA move about in solution, the sequences that are more highly abundant have a greater probability of finding a matching complementary sequence with which to re-hybridize. Thus, as re-hybridization reaches its limit, the remaining single stranded cDNA represents a normalized sequence library.

The difference between the two methods lies in their approach for isolating the single stranded cDNA library from the re-hybridized double stranded cDNA molecules.

In the DSN method, an enzyme which specifically cleaves double stranded DNA is used to decompose all double stranded cDNA within the solution. The solution is then purified and size-selected for cDNA sequences above a certain length. These sequences are then amplified using the Polymerase Chain Reaction (PCR).

In the column method, the denatured and re-hybridized cDNA library is passed through a heated column filled with hydroxyapatite granules. The hydroxyapatite preferentially binds to larger DNA molecules. The size of DNA that is bound is controlled by the concentration of phosphate buffer in which the cDNA library is dissolved. Thus the concentration of phosphate buffer must be tuned specifically for cDNA molecules within a certain range of sequence length. The cDNA is eluted through the column using increasing concentrations of phosphate buffer to extract increasing sizes of DNA molecules. Since the single stranded cDNA will be roughly one half the size of the re-hybridized cDNA, elution of the single stranded fraction can be managed if the mean cDNA sequence length is known. The resulting elution is intended to be enriched for the single stranded cDNA which are then amplified using PCR.

In both the DSN and column methods, known adapters must be attached to the ends of the cDNA prior to normalization to facilitate PCR amplification (so that appropriate primers can be used).

Since both methods are subtractive by nature with the depletion of large fractions of cDNA, the amount of starting cDNA is typically required to be higher than 1µg for the DSN approach and 4µg for the column approach.

Since the DSN method uses enzymes which cleave all double stranded cDNA, in theory it can deplete low abundance sequences with segments that match high abundance sequences. This effect can also increase the probability of forming PCR chimeras. PCR chimeras are formed when incomplete single stranded cDNA sequences act as primers to other sequences thus combining the sequences in a way that does not occur in nature. PCR chimeras represent false positives for novel isoforms and are extremely challenging to distinguish from true alternative isoforms. Validating PCR chimeras typically requires in-depth biochemical assays.

Since the column method only allows for segregation of high abundance and low abundance fractions within a narrow size range, it has significant bias against longer cDNA sequences. The effect of this is a loss of representation for longer RNA sequences.

### Column method

As described above the hydroxyapatite column method relies on the denaturation and re-hybridization of cDNA strands. As the single stranded cDNA move about in solution, the sequences that are more highly abundant have a greater probability of finding a matching complementary sequence with which to re-hybridize. In the column method, the denatured and re-hybridized cDNA library is passed through a heated column filled with hydroxyapatite granules. The hydroxyapatite preferentially binds to larger DNA molecules. Since the single stranded cDNA will be roughly one half the size of the re-hybridized cDNA, elution of the single stranded fraction can be managed if the mean cDNA sequence length is known. The resulting elution is intended to be enriched for the single stranded cDNA which are then amplified using PCR.

The hydroxyapatite column method was carried out as described in Andrews-Pfannkoch et al. (Andrews-Pfannkoch, C., Fadrosh, D. W., Thorpe, J. & Williamson, S. J. Hydroxyapatite-mediated separation of double-stranded DNA, single-stranded DNA, and RNA genomes from natural viral assemblages. Appl. Environ. Microbiol. 76, 5039-5045 (2010) with 4µg cDNA starting sample. The hydroxyapatite column method did not produce usable yield when used with 2µg or less of cDNA.

As the column method is based on separation by size this method results in a loss of representation for longer RNA sequences (longer than 4kb) which is observable in the length distribution before and after normalization.

### DSN method

As for the column method, the DSN method relies on the denaturation and re-hybridization of cDNA strands. As the single stranded cDNA move about in solution, the sequences that are more highly abundant have a greater probability of finding a matching complementary sequence with which to re-hybridize. In the DSN method, an enzyme which specifically cleaves double stranded DNA is used to decompose all double stranded cDNA within the solution.

The commercially available Evrogen Trimmer-2 cDNA normalization kit uses the DSN method. This kit was used according to the manufacturer's instructions with 1µg cDNA starting sample. However, to produce enough material for long read RNA sequencing it was found necessary to use 2µg of cDNA.

The DSN method was found to completely eradicate high abundance RNAs and this would also be expected to be the case for RNAs with sequence similarity to the high abundance RNAs. Thus, over-depletion was observed in which high abundance RNAs were not just reduced in quantity but were completely removed from the samples. Table 1 illustrates over-depletion of ranks 1-20 and shows a selection of lower ranks (55, 64, 77, 92 and 98) in which the RNAs were significantly reduced but not completely depleted.

**Table 1 - over-depletion of high abundance RNAs with DSN method**

| Rank | Ensembl Transcript ID | Standard Read Count | DSNase Read Count |
|---|---|---|---|
| 1 | ENSGALT00000023323 | 18937 | 0 |
| 2 | ENSGALT00000039524 | 14928 | 0 |
| 3 | ENSGALT00000052417 | 9147 | 0 |
| 4 | ENSGALT00000022187 | 4293 | 0 |
| 5 | ENSGALT0000001 1687 | 3822 | 0 |
| 6 | ENSGALT00000041222 | 3610 | 0 |
| 7 | ENSGALT00000016648 | 2996 | 0 |
| 8 | ENSGALT00000044106 | 2949 | 0 |
| 9 | ENSGALT00000084298 | 2825 | 0 |
| 10 | ENSGALT00000023065 | 2677 | 0 |
| 11 | ENSGALT00000088434 | 2255 | 0 |
| 12 | ENSGALT00000011524 | 2151 | 0 |
| 13 | ENSGALT00000055441 | 2127 | 0 |
| 14 | ENSGALT00000044027 | 2080 | 0 |
| 15 | ENSGALT00000078059 | 2052 | 0 |
| 16 | ENSGALT00000084500 | 2020 | 0 |
| 17 | ENSGALT00000071026 | 1839 | 0 |
| 18 | ENSGALT00000015018 | 1742 | 0 |
| 19 | ENSGALT00000046070 | 1731 | 0 |
| 20 | ENSGALT00000014652 | 1718 | 0 |
| 55 | ENSGALT00000066490 | 649 | 1 |
| 64 | ENSGALT00000087166 | 630 | 5 |
| 77 | ENSGALT00000072914 | 565 | 1 |
| 92 | ENSGALT00000043160 | 475 | 1 |
| 98 | ENSGALT00000070660 | 451 | 2 |

In addition, the DSN method creates conditions in which artificial chimeric sequences can be generated, which can show up as false positives for gene predictions.

### Selective amplification method (Level-up)

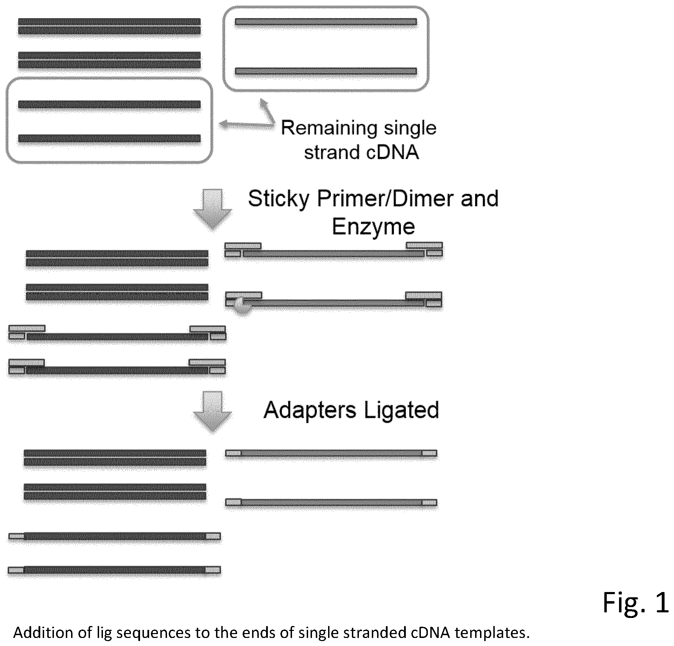

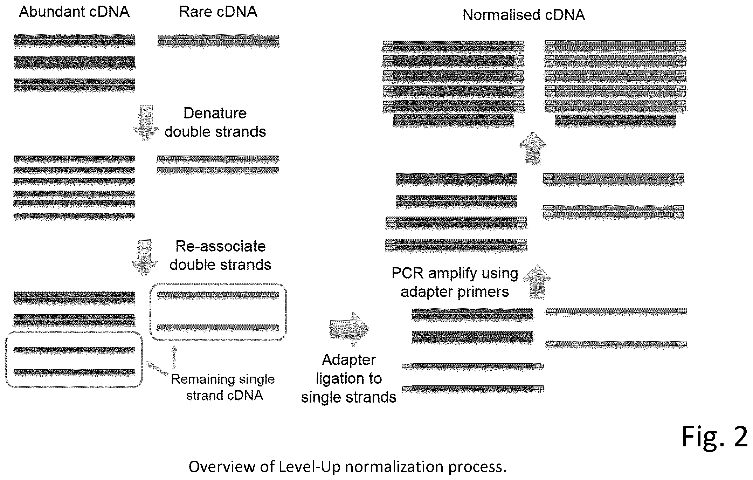

This method is illustrated in Figures 1 to 3. In overview, with reference to Figure 2, a cDNA sample, comprising cDNA having known 5' and 3' pre-attached adapters, is denatured to provide a single stranded cDNA sample; the sample is then re-hybridised or re-associated to provide a mixture of single and double stranded cDNA. This single stranded cDNA represents the low abundance cDNA. The single stranded cDNA within the re-associated sample is then modified by adding oligonucleotides to the 5' and 3' pre-attached adapters. The cDNA sample is amplified using primers to these oligonucleotides. This process selectively increases the content of only the single stranded cDNA within the re-associated sample, thereby increasing the content of the low abundance cDNA in the sample, thereby providing a normalized sample.

The input cDNA library for the selective amplification method is double-stranded and the double stranded templates each comprise a 5' pre-attached adapter of known nucleotide sequence and a 3' pre-attached adapter of known nucleotide sequence. Since the present method is an additive method, lower quantities of starting cDNA are required as compared to prior art normalization methods. In the DSNase method, a minimum of 1µg of input cDNA is required and in the column method a minimum of 4µg of input cDNA is required. In testing, it was found that the present method could be applied with as little as 20ng of starting cDNA.

The input cDNA is combined with a hybridization buffer and the solution heated to denaturation temperatures - which is about 98 degrees Celsius, to produce denatured single stranded cDNA templates. After 5-10 minutes, the solution is then brought down to re-hybridization temperature - about 68 degrees Celsius. The solution is incubated at this temperature for between 0-24 hours depending on the amount of normalization required, for example 3-10 hours. 7 hours is a typical duration for the re-association step. This step produces a re-associated or re-hybridised sample comprising post-association double stranded cDNA templates and post-association single stranded cDNA templates.

After incubation, oligonucleotide dimers (termed K-linkers), of the present invention are added. These oligonucleotide dimers are discussed in more detail below. At this point, the solution can be left to incubate at 68 degrees Celsius from 0-1 hour. 5 minutes is a typical duration for this incubation step. The solution is then brought down to the annealing temperatures of the K-linkers, which is typically between 40-60 degrees Celsius, for example 44 degrees celsius. The solution is incubated at the temperature from between 10 minutes-2 hours, for example 25 minutes. This step anneals the K-linkers to the post-association single stranded cDNA templates.

After this incubation period, DNA ligase is added together with ligation mix. The solution is incubated at this same temperature for 0.5-2 hours, for example 1 hour, and then brought down to room temperature. This step results in formation of ligated-adapter-cDNA templates where an oligonucleotide from a K-linker is ligated to each end of a post-association single stranded cDNA template. At this point, the cDNA may be purified (for example using Pronex or Ampure beads) or may be used directly for PCR amplification using primers based on the K-linker sequences.

After PCR amplification, the cDNA is then purified using any appropriate means and the resultant cDNA represents the normalized cDNA library.

The post-association double stranded cDNA templates can be removed before PCR amplification but, after testing, the inventor has shown that leaving the double stranded cDNA in the solution does not negatively impact the normalization process.

Indeed the post-association double stranded cDNA can also be analysed and used, for example, to attain estimates of gene expression. This involves an additional (PCR) selective amplification step using primers to the known 5' pre-attached adapter and known 3' pre-attached adapter, wherein molecular barcodes are included in the primers. PCR amplification using primers based on the K-linker sequences is carried out first followed by a single PCR cycle using the primers to the known 5' pre-attached adapter and known 3' pre-attached adapter. The PCR can be paused to add the further primers for the final cycle. Alternatively, the cDNA can be purified after the PCR using the primers based on the K-linker sequences and a new PCR carried out for a single cycle using the primers to the known 5' pre-attached adapter and known 3' pre-attached adapter. In both cases the product will be a mixture of two distinguishable fractions comprised of sequences originating from the post-association double stranded cDNA templates and from the post-association single stranded cDNA templates. The molecular barcode addition allows for the identification of the source molecule during sequencing analysis. This aspect of the invention may be used in multiplex sequencing.

### Design of oligonucleotide dimer complexes

The oligonucleotide dimer compositions of the present invention comprise front and back oligonucleotide dimers (front and back K-linkers), which both anneal to the same strand of post-association single stranded cDNA.

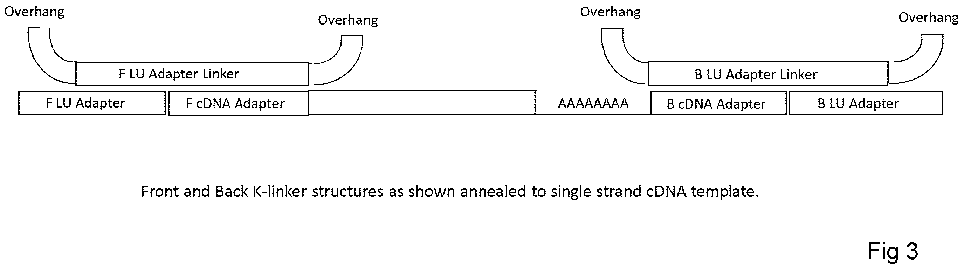

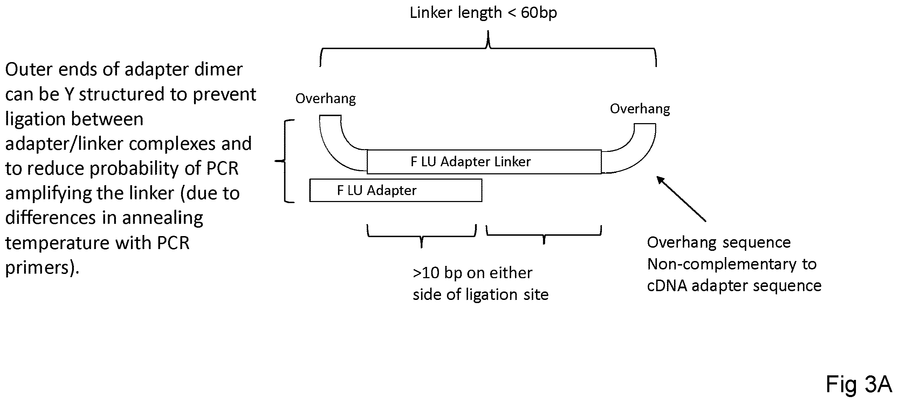

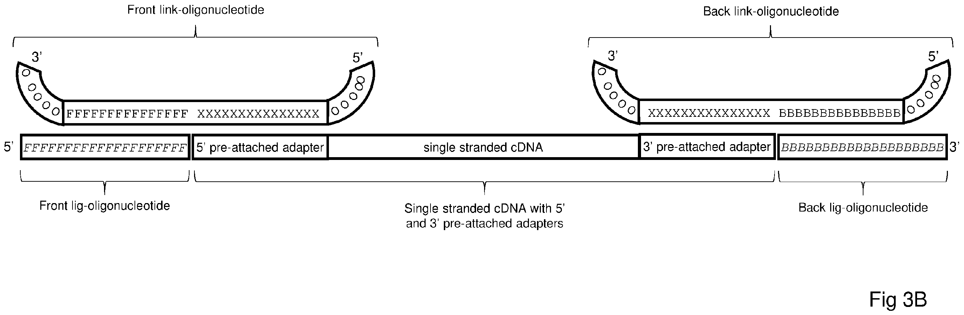

With reference to Figures 3 and 3A, each K-linker comprises two oligonucleotide sequences; one is termed the link-oligonucleotide (also termed 'link'; termed `LU adapter linker' in Figures 3 and 3A)) and the other is termed the lig-oligonucleotide (also termed 'lig'; termed `LU adapter' in Figures 3 and 3A).

The link sequence includes a region complementary with the known 3'/5' pre-attached adapter sequence (that was previously added to the cDNA) and a region complementary with the lig.

As also depicted in Figures 3 and 3A, the link can be designed to have an overhang region at one end which is non-complementary the known pre-attached adapter sequences, this region is termed a 'template overhang'. The opposite end of the link can be provided with a similar overhang that is non-complementary to the lig sequence, this is termed a `lig overhang' or `lig-oligonucleotide overhang'.

The purpose of the link is to anneal to both the pre-attached adapter of the post-association single stranded cDNA template and the lig, in such a way that, in use, one end of the cDNA template is adjacent one end of the lig. By positioning the cDNA template and the lig in this way, DNA ligase can be used to ligate the cDNA template to the lig, thus adding the lig sequence to the end of the cDNA template. A lig sequence is added to both the 5' and 3' ends of the single stranded cDNA template. Front and back K-linkers are used to add these ligs to the 5' and 3' ends of the cDNA template respectively.

In the method described above, once a lig has been added to each end of the single stranded cDNA template, primers based on the sequences of the added ligs are used to selectively amplify the single stranded cDNA fraction that has successfully ligated to both front and back lig sequences. In this way, PCR can be used to amplify only the low abundance post-association single stranded cDNA fraction.

The particular structure of the K-linkers provides advantages to overall normalization performance with the front K-linker and the back K-linker having different functions provided by their specific structural characteristics.

The front K-linker which binds to the 5' end of the single strand cDNA template (shown in the figures as the reverse complement to the original RNA sequence) can be designed so that the K-linker does not act as a primer during PCR amplification.

To provide additional advantages, the front K-linker does not have a blunt end on the lig side to allow for the use of DNA ligase that can perform blunt end ligation in the selective amplification process. Providing the lig side of the K-linker complex with a non-blunt end also avoids ligation to other K-linker complexes or to the double stranded cDNA in the solution.

Since the front linker has a 5' to 3' directionality pointing away from the template, the linker itself cannot act as a primer of the template. However, in some instances, the front lig or PCR primers could potentially anneal to the linker during PCR amplification and undergo polymerase extension to take on the template side sequence of the link. Providing the link with an overhang on the template side avoids the extended lig/primer acting as a primer for the cDNA sequences which do not have the lig sequences added to their ends, i.e., the sequences that are high abundance. Accordingly, a template overhang structure for the back link can be provided.

The back K-linker which binds to the 3' end of the single stranded cDNA template (shown in the figures as the reverse complement to the original RNA sequence) can be designed so that the K-linker does not act as a primer during PCR amplification. This can be achieved using the template overhang, which is described above and illustrated in Figures 3 and 3A.

The back K-linker can be provided without a blunt end on the lig side for the same reason that the front K-linker can be designed to have an overhang on the lig side. If the lig side of the back K-linker complex had a blunt end, in some instances, the lig could potentially be ligated to other K-linker complexes or to the double stranded cDNA in the sample, which could result in linking of cDNA templates.

The overhangs also serve to lower the annealing temperature of the K-linker complexes so that they are less likely to act as primers for each other during PCR amplification, where higher annealing temperatures are used. Overhangs may reduce unintended priming. Additionally or alternatively, overhangs may provide an indicator that enables the measurement of the amount of unintended priming. For example, if the K-linker complexes with their overhangs are able to prime a template then it will be possible to see the overhang sequence in the sequencing data and to conclude that it was a product of un-intended priming.

All overhangs should ideally be between about 1bp and about 20bp, preferably 3bp. Longer overhangs could be used but it would make the design more difficult since there are fewer sequence compositions that would prevent unintended priming as the overhangs get longer.

The complementary regions between the cDNA template and the link, and between the link and the lig should be long enough for annealing at the temperature of activity for the ligase to be used. Nick repair ligases are particularly preferred for this process which typically require about five or more complementary bases on either side of the ligation site.

The combined length of the link and corresponding lig should ideally be less than about 300bp, preferably less than about 200bp, to reduce carry over during the purification process.

### Example sequence representations:

All example sequence structures are provided in 5' to 3' orientation. Also illustrated in Figure 3B.
Front link:
   OOOOOXXXXXXXXXXXXXXXFFFFFFFFFFFFFFFOOOOO
Front lig:
   *FFFFFFFFFFFFFFFFFFFF*
Back link:
   OOOOOBBBBBBBBBBBBBBBXXXXXXXXXXXXXXXOOOOO
Back lig:
   *BBBBBBBBBBBBBBBBBBBB*
X- Nucleotides complementary to 5' / 3" pre-attached adapter
O - Overhang sequences
F - Sequence complementary to front lig to be ligated
*F* - Sequence of the front lig
B - Sequence complementary to back lig to be ligated
*B* - Sequence of the back lig

### Example of oligonucleotides and single stranded cDNA template used in the selective amplification method:

### Primer sequences from NEB/PacBio cDNA synthesis kit

Iso-Seq Express Fwd:
   GGCAATGAAGTCGCAGGGTTG
Iso-Seq Express Rev:
   AAGCAGTGGTATCAACGCAGAG
Front Link:
   ATAGCGTTGATACCACTGCTT**CTCACGACAGACTCGC**TAA
Front Lig and Primer:
   TGGACTGAT ***GCGAGTCTGTCGTGAG***
Back Link:
   AAT**GACGCTGGACGAACAC** GGCAATGAAGTCGCAG ACA
Back Lig:
   ***GTGTTCGTCCAGCGTC*** CAGGTGAGTGG
Primer:
   CCACTCACCTG **GACGCTGGACGAACAC**

Overhangs are shown underlined. Regions of complementarity between oligonucleotides are shown in bold.

### Single stranded cDNA template:

**AAGCAGTGGTATCAACGC**AGAGNNNNNNNNNNNNNNNNCAACC**CTGCGACTTCATTG CC** (i.e. 5'-3' - sequence of 5' pre-attached adapter, sequence of cDNA represented by N and sequence of 3' pre-attached adapter; regions of complementarity to front link and back link-oligonucleotides are shown in bold).

### Preparation of oligonucleotide dimers

Once designed, the dimers can be prepared using standard techniques well known in the art.

### Amplification of oligonucleotide-cDNA templates

After ligation of the lig to the 3' and 5' ends of the cDNA template, the resulting solution can be purified for cDNA using an appropriate cDNA purification method. The purification step may be skipped, but skipping may result in lower efficiency for PCR amplification.

After purification or after ligation the resulting material can be PCR amplified using forward and reverse primers based on the lig sequence. The primer sequences can be chosen to have a higher annealing temperature than the complementary regions of the template/link/lig to avoid unwanted priming.

If required, the number of optimal PCR cycles can be identified by first running a qPCR experiment to identify the inflection point of the amplification curve.

The resulting cDNA from PCR amplification can then be purified and used as input for any downstream processes, such as sequencing.

### Validation of the selectively amplified sample

The effect of selective amplification or normalization can be indirectly measured by measuring the length distribution of the cDNA library using gel electrophoresis or directly measured using sequencing.

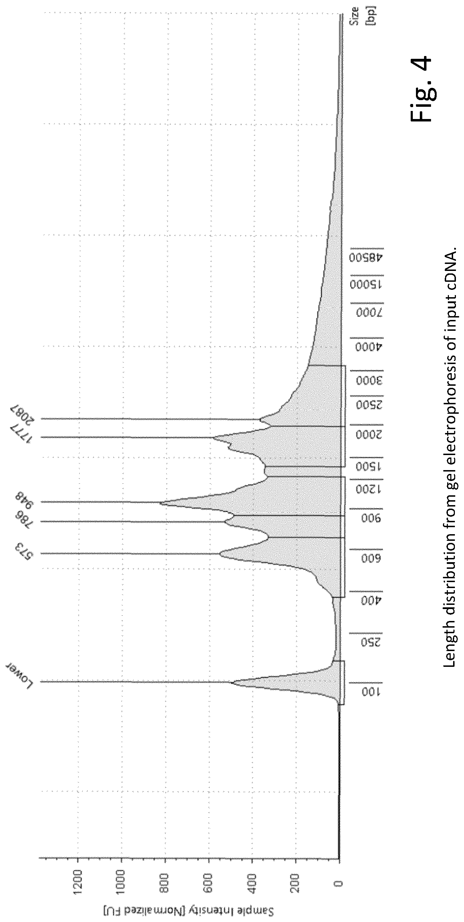

To identify the effect of normalization using gel electrophoresis, the length distribution plots of the input cDNA can be compared to the normalized cDNA. The input cDNA will typically show peaks along the length distribution which correspond to high abundance transcript sequences (Figure 4).

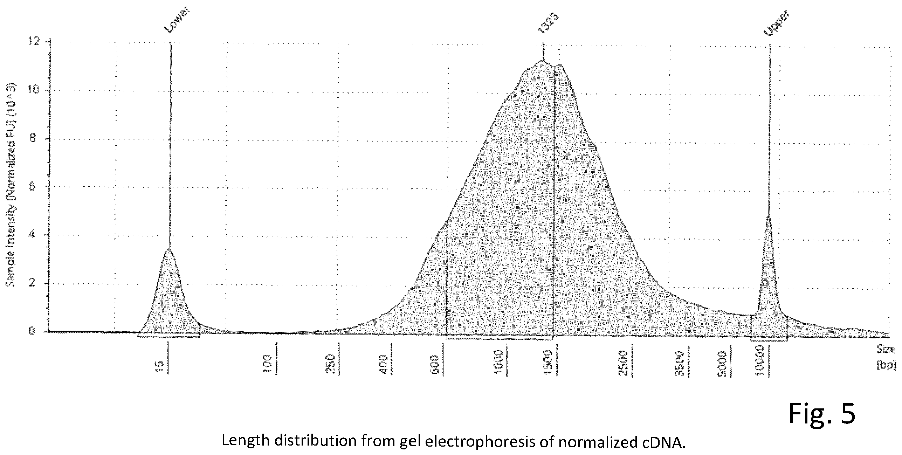

The normalized cDNA will have a length distribution that resembles a normal distribution with no sharp peaks (Figure 5). This represents a uniform distribution across transcript sequences.

When using sequencing for direct measurement of normalization, the preferred method of sequencing is long read sequencing. This allows for the identification of distinct isoforms. The results for direct measurement can be either a plot of the number of reads per gene or a saturation plot showing the number of new genes identified with increase depth of sequencing.

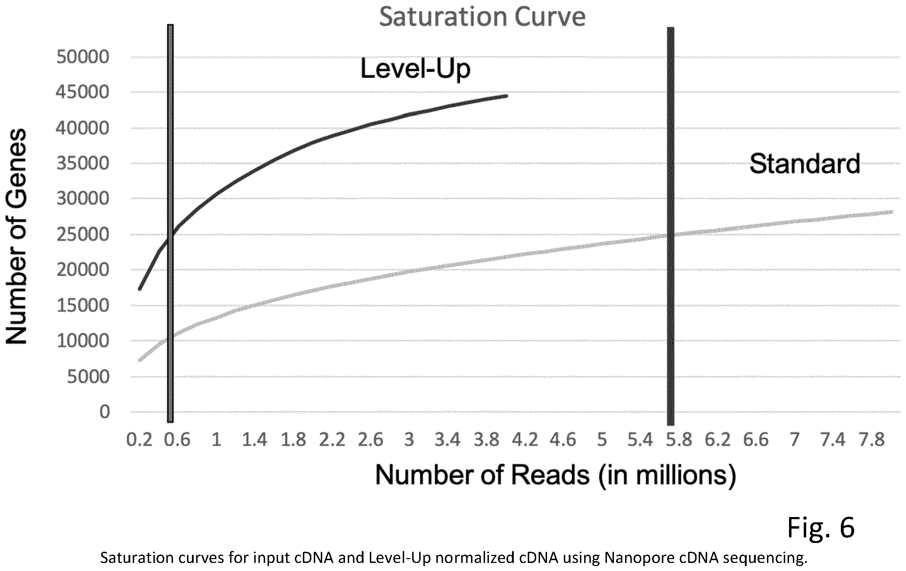

To validate the present method, the inventors performed Nanopore cDNA sequencing on both the input cDNA library and a cDNA library generated by the present method. They compared the saturation curves for each of the libraries (Figure 6). The curve for the present method is higher than the curve for the input cDNA by multiple factors. This indicates a more uniformly distributed cDNA library.

### Applications of selective amplification

A primary application for the present method of selective amplification is for improving the discovery and detection of low abundance genes and isoforms. When combining the present method with sequencing, the sampling efficiency is increased for identifying all unique genes within a sample.

The present method can be applied to any double stranded cDNA library with known adapters on the ends and lengths that can be amplified via the PCR method. This means that it can be used in DNA sequencing.

Another important aspect of the present method is the ability to select cDNA having known lig sequences. This aspect could be applied for single cell sequencing where adapters are necessary for assigning reads to individual cells. In this application, cDNA sequences without cell identifying barcodes/adapters arise within the cDNA library. These are known as template switching oligo (TSO) artefacts and are undesirable in single cell sequencing projects due to not being assignable to a cell of origin. The present method can be applied with a short re-hybridization step to only select for cDNA sequences with the desired lig sequences thus effectively limiting the sequencing of TSO artefacts. The present method can also be performed with single cell cDNA libraries to both remove TSO artefacts and to improve transcriptome coverage per cell.

### Discussion

The present method of selective cDNA amplification represents an innovative method of achieving greater non-targeted discovery/detection of low abundance nucleic acids. Being non-targeted it is not necessary to know which sequences are of low or high abundance in a given sample.

It differs from existing normalization approaches by using an additive method where the current approaches use a depletion method. The additive method allows for the present method to be used with significantly smaller amounts of starting cDNA. It aids the detection of low abundance nucleic acids and provides for greater confidence in determining the absence of particular nucleic acids. The additive method also prevents over depletion and artificial chimerization, which is endemic to the DSNase method. The present method is also only length biased to the degree that PCR amplification is length biased. Thus, it can be run successfully with longer cDNA molecules.

The example systems, methods, and acts described in the embodiments presented previously are illustrative, and, in alternative embodiments, certain acts can be performed in a different order, in parallel with one another, omitted entirely, and/or combined between different example embodiments, and/or certain additional acts can be performed, without departing from the scope and spirit of various embodiments. Accordingly, such alternative embodiments are included in the examples described herein.

Although specific embodiments have been described above in detail, the description is merely for purposes of illustration. It should be appreciated, therefore, that many aspects described above are not intended as required or essential elements unless explicitly stated otherwise.

Modifications of, and equivalent components or acts corresponding to, the disclosed aspects of the example embodiments, in addition to those described above, can be made by a person of ordinary skill in the art, having the benefit of the present disclosure, without departing from the spirit and scope of embodiments defined in the following claims, the scope of which is to be accorded the broadest interpretation so as to encompass such modifications and equivalent structures.

### EXAMPLES

The present invention will be further understood by reference to the following experimental examples.

### EXAMPLE 1

### Using a novel transcriptomic discovery platform to detect unique RNA signatures in epithelial cancers

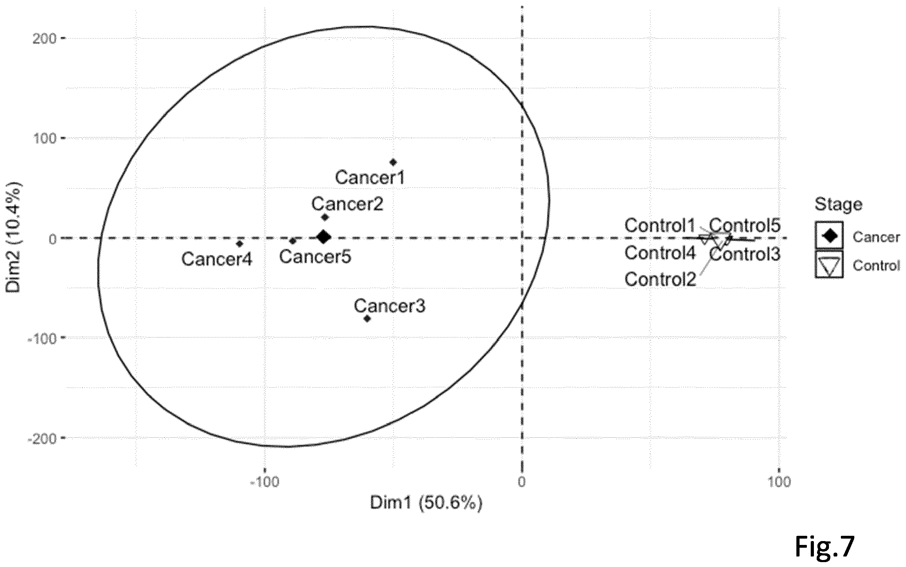

The inventor has validated the use of Level-up with RNA from cancer cell lines and blood samples. In these initial studies thousands of previously un-annotated isoforms which have the potential to be used as cancer biomarkers have been found. Figure 7 shows the clustering of cancer samples (blood samples taken from people who have been diagnosed with breast cancer) and control samples (blood samples taken from people who have not been diagnosed with breast cancer) based on 13573 transcripts identified in these initial studies. The cancer samples can be readily distinguished from the control samples.

An example protocol is provided for discovery of unique RNA signatures in epithelial cancers:

### Stage 1

Blood handling validation is performed using 5 samples from 5 individuals (25 total samples). Blood is collected and stored in PAXgene RNA blood tubes (each with a blood capacity of 2.5 ml). After collection the tubes are transported on dry ice the same day from the local collection centre.

For each biological replicate the 5 collected tubes are processed according to different simulated handling situations. These include:
1. Processing tube on the same day as collection.
2. Processing tube 72 hours after collection.
3. Processing tube 2 weeks after collection.
4. Processing tube 4 weeks after collection.
5. Storing tube long term at -80C to be processed in 4-12 months.

RNA is extracted from the PAXgene tubes using a Qiagen PAXgene Blood RNA Kit (Cat. No. / ID: 762164). The resulting RNA is converted into cDNA using the NEBNext^{®} Single Cell/Low Input cDNA Synthesis & Amplification Module (E6421S). The resulting cDNA is processed by normalization as described herein. The normalized cDNA library will then be sequenced using an Oxford Nanopore Technologies Minion MK1C sequencer.

### Stage 2

Having optimised sample processing and assured quality of data generated, the study then progresses to patient samples.

### Inclusion and exclusion criteria

Inclusion criteria: All female patients who are pre-menopausal, over 18 years of age but under 50 years with invasive breast cancer or a benign condition (B2).

Exclusion criteria: History of any cancer or a concurrent cancer of other type, auto-immune diseases, and women not able or willing to give informed consent.

### Identification and recruitment of patients to the prospective cohort study

Eligible patients are identified from multidisciplinary team (MDT) meetings, with the results of triple assessment. When patients attend for results at the clinic, with the breast surgeon, they are invited to participate in the study and given a patient information sheet (PIS). Patients given a PIS will be followed up by the research team after 72 hrs. If the patient elects to participate in the study, they meet with the research nurse and sign a consent form or be offered to be consented remotely.

### Anonymisation

Once enrolled in the study, the research nurse assigns the patient with a unique ID number that is linked through a secure database to the background data and used therein to collect and identify samples.

### Background data collection

The following data is collected for each patient: Baseline demographic, menopausal status, past medical history, medication history, drug and alcohol consumption, BMI and family history.

Triple assessment including presentation, examination findings, imaging, and biopsy results is collated. Post-surgical histology is also be collected. Any additional prognostic information, such as use of biomolecular assays is also collected.

### Blood sample collection

On the morning of surgery, a blood sample is obtained and stored at -20°C to be transported to the processing centre. For any patient who is not proceeding to surgery e.g. fibroadenoma, they are invited to attend the day unit where blood tests are taken by the same team. The sample size is 20ml with a maximum collection that day of 30ml.

### Processing of blood samples

Blood samples are processed from the collection tubes in a laminar flow class 2 safety cabinet using an RNA extraction kit provided by Qiagen. After RNA has been extracted, the remaining solid waste is autoclaved and discarded using a supplier of human medical waste removal services. Liquid waste will be decontaminated and disposed as per University of Edinburgh Guidance on use of human samples. Blood samples that are not processed on the same day of arrival are stored in a secure -80°C freezer. A sealed container is used to transport blood samples between the freezer and the safety cabinet.

### Data analysis

Transcriptomics data is stored and processed in secure Amazon Web Services cloud repositories and servers. There is no personal information stored in the same computational location.

Oxford Nanopore Technologies (ONT) Minion sequencing machines are used to run cDNA sequencing on the samples. This outputs raw data as fast5 files. The most up-to-date high accuracy basecaller from ONT (Bonito) is run to convert to fastq sequence files. The nanopore reads are filtered for quality using seqkit and adapters removed using pychopper. The trimmed reads are then mapped to the HG38 (or newer version) human reference genome assembly using Minimap2.

### Stages 3-4

The study is amended to initially increase the sample size and subsequently extend into colorectal and ovarian cancer.

### Statistical considerations and sample size

For stage 1 of the study, 5 samples are processed. This is sufficient to produce technical and biological replicates and undertake initial quality assessment.

Data handling and processing are performed as in stage 2.

For stage 2 of the study, initially 30 samples from benign and 30 from cancer patients are collected and processed. This data will inform the subsequent sample sizes for stages 3 and 4 of the overall study.

### Ethical approval

Ethical approval is sought from the National Research Ethics Service prior to the initiation of the study.

### Informed Consent process

Written informed consent is obtained from all patients participating in this study to collect their demographic and clinical data and for the blood sample and subsequent storage of the transcriptomic material and data. Where patients elect to do so, they are consented over the telephone. Patients can withdraw consent at any point. Anyone not deemed able to give informed consent are excluded from the study.

### Quality control

In order to assess that samples were labelled correctly and that there are no handling issues with each sample, an in silico quality control test is performed. This test is made up of checks for known genes that should be present in all samples as well as clustering analysis to assess outliers which could represent sample contamination.

Genes present in the sample data that should not be present are also looked for. For example, genes from other species.

### EXAMPLE 2

### Blood sample collection procedure for full-length RNA extraction

cDNA is DNA synthesized from a RNA template. Thus, the quality of the cDNA sample is related to the RNA from which it is reverse transcribed. Prior art processes for handling blood samples prior to RNA extraction involve overnight thawing of the frozen blood samples. The inventor has found that this leads to significant RNA degradation which negatively impacts long-read sequencing. The inventor uses the following protocol to process blood samples prior to RNA extraction in order to minimize degradation and optimize RNA extraction for long-read sequencing.

Blood sample collection procedure for full-length RNA extraction
- Draw 2.5 ml of blood into the PAXgene Blood RNA Tube at room temperature (18-25°C). Gently invert the blood tube 10 times immediately after blood collection.
- If RNA is to be extracted on the same day as sample collection, store the blood sample upright at room temperature (18-25°C) for 2-3 hours, then continue with RNA extraction immediately using the PAXgene Blood RNA Kit.
If RNA extraction is not carried out on the day of blood collection, follow instructions below for sample freezing, storage, and thawing:
- The blood sample should be stored at -20°C or below immediately after collection. For long-term storage, freeze the blood sample at -20°C for 24 hours before transferring to a -70°C or -80°C freezer.
- If the sample is to be transferred to a different location, ship on dry ice to make sure the sample stays frozen during transportation.
- On the day of RNA extraction, thaw the blood sample by placing the sample tube upright on a rack and incubating at room temperature (18-25°C) for 1-3 hours. Once the blood is fully thawed, gently invert the sample tube 10 times, incubate at room temperature for another 2 hours, then perform RNA extraction immediately using the PAXgene Blood RNA Kit.

As shown in Table 2 RNA integrity is improved with a shorter (3-hour) thawing period relative to overnight thawing. RNA Integrity Number was calculated as in Schroeder et al. (The RIN: an RNA integrity number for assigning integrity values to RNA measurements. BMC Molecular Biology 7, 3 (2006). https://doi.org/10.1186/1471-2199-7-3.) Samples were placed at -20°C within 12 hours for the "-20°C 2 weeks; overnight thawing" and "-20°C 1 month; 3-hour thawing" tests (second and third tests). 5 individuals were used for each test. All thawing was carried out at room temperature.

**Table 2 - blood sample storage conditions and RNA integrity**

| **Blood sample storage Condition** | **RNA Integrity Number** |
|---|---|
| Fresh | 8.3 ± 0.2 |
| -20°C 2 weeks; overnight thawing | 7.8 ± 0.2 |
| -20°C 1 month; 3-hour thawing | 8.4 ± 0.3 |
| 4°C 3 days, then -20°C 2 months; 3-hour thawing | 8.0 ± 0.3 |

The present invention is not to be limited in scope by the specific embodiments described herein. Indeed, various modifications of the invention in addition to those described herein will become apparent to those skilled in the art from the foregoing description and accompanying figures. Such modifications are intended to fall within the scope of the appended claims. Moreover, all embodiments described herein are considered to be broadly applicable and combinable with any and all other consistent embodiments, as appropriate.

Various publications are cited herein, the disclosures of which are incorporated by reference in their entireties.

## Claims

1. A method for producing an RNA vaccine for a subject with a disease, the method comprising:
(i) providing an RNA sample extracted from a blood sample obtained from the subject;
(ii) processing the RNA sample, optionally comprising synthesizing cDNA using the RNA as a template;
(iii) sequencing the processed RNA or cDNA, wherein the sequencing output is used to identify the presence of an RNA transcript sequence associated with the disease; and
(iv) using the identified RNA transcript sequence to produce a first RNA vaccine for the subject.

2. The method of claim 1 further comprising:
(v) providing a further RNA sample extracted from a blood sample obtained from the subject at a later time point;
(vi) processing the RNA sample, optionally comprising synthesizing cDNA using the RNA as a template;
(vii) sequencing the processed RNA or cDNA, wherein the sequencing output is used to identify the presence of an RNA transcript sequence associated with the disease, and
(viii) using the identified RNA transcript sequence to produce a further RNA vaccine for the subject.

3. The method of claim 1 or claim 2, wherein using the identified RNA transcript sequence to produce the RNA vaccine comprises producing an RNA molecule comprising at least a portion of the sequence, wherein the RNA molecule comprises an open reading frame (ORF) encoding at least one antigenic peptide.

4. The method of claim 3, wherein the RNA molecule further comprises a 5' UTR, 3' UTR, a polyA tail and/or a 5' cap.

5. A method for producing a database of disease biomarkers, the method comprising:
(i) providing two or more RNA samples extracted from blood samples obtained from one or more subjects with a disease;
(ii) processing the RNA samples, optionally comprising synthesizing cDNA using the RNA as a template;
(iii) sequencing the processed RNA or cDNA, wherein the sequencing output is used to identify two or more disease biomarkers; and
(iv) compiling the disease biomarkers to form a database.

6. A method for discovering a biomarker for an autoimmune disease comprising:
(i) providing a first RNA sample extracted from a blood sample obtained from a subject with the disease and a second RNA sample extracted from a blood sample obtained from a subject without the disease;
(ii) processing the first and the second RNA samples, optionally comprising synthesizing cDNA using the RNA as a template;
(iii) sequencing the processed RNA or cDNA; and
(iv) comparing the sequencing output for the first and second samples to discover a disease biomarker.

7. A method for diagnosing an autoimmune disease in a subject comprising:
(i) providing an RNA sample extracted from a blood sample obtained from the subject;
(ii) processing the RNA sample, optionally comprising synthesizing cDNA using the RNA as a template; and
(iii) sequencing the processed RNA or cDNA, wherein the sequencing output is used to identify whether the subject has the disease.

8. The method of claim 7, wherein the sequencing output is used to identify whether the subject has the disease by comparing to a database of biomarkers, optionally wherein the database of biomarkers is produced using the method of claim 5.

9. The method of any previous claim, wherein processing the RNA sample(s) comprises reducing the variability in the levels of different RNA or cDNA sequences in the sample(s).

10. The method of any previous claim, wherein in the processed RNA or cDNA the relative abundance of all the unique RNA or cDNA sequences is more equal.

11. The method of any previous claim, wherein processing the RNA sample(s) improves detection of low abundance RNA or cDNA, optionally wherein processing the RNA sample(s) comprises increasing the amount of low abundance RNA or cDNA within each sample.

12. The method of any previous claim, wherein the RNA is full-length RNA.

13. The method of any previous claim, wherein sequencing comprises the use of long read sequencing.

14. The method of any of claims 6 to 13, wherein the autoimmune disease is arthritis, celiac disease, diabetes mellitus type 1, graves' disease, inflammatory bowel disease, multiple sclerosis, alopecia areata, Addison's disease, pernicious anemia, psoriasis, systemic lupus erythematosus, myasthenia gravis, Hashimoto's thyroiditis, Vitiligo, Sjogren's syndrome, myositis, chronic inflammatory demyelinating polyneuropathy (CIDP), dermatomyositis, Guillain-Barre syndrome, ulcerative colitis and/or vasculitis.

15. The method of any of claims 1 to 4 or 9 to 13, wherein using the sequencing output to identify the presence of a sequence associated with the disease comprises identifying an RNA transcript, which encodes a protein isoform present in subjects with the disease.
